# EUROPEAN PATENT APPLICATION

(11) **EP 4 495 222 A2**
(43) Date of publication of application: **22.01.2025**
(21) Application number: 24218176.6
(22) Date of filing: 11.01.2017
(51) Int. Cl.: C12M 1/42

(54) **INTRACELLULAR DELIVERY OF COMPLEXES**

(30) Priority: 12.01.2016 US 201662277858 P
(62) Divisional of application: 17704337.9
(71) Applicant: SQZ Biotechnologies Company, Watertown, MA 02472 (US)
(72) Inventor: DITOMMASO, Tia, MA 02472, Watertown (US); BERNSTEIN, Howard, MA 02472, Watertown (US); SHAREI, Armon, MA 02472, Watertown (US); GILBERT, Jonathan, MA 02472, Watertown (US)
(74) Representative: Wilson, Justin Scott

(57) **Abstract**

The present invention provides methods for delivering a transient and/or reversible complex into a cell including passing a cell suspension through a constriction, wherein said constriction deforms the cell, thereby causing a perturbation of the cell such that the complex enters the cell.

## Description

### CROSS-REREFENCE TO RELATED APPLICATIONS

This application claims priority to U.S. Provisional Application No. 62/277,858, filed on January 12, 2016, which is hereby incorporated by reference in its entirety.

### FIELD OF THE INVENTION

The present disclosure relates generally to methods for delivering a complex into a cell by passing a cell suspension through a constriction.

### BACKGROUND

Intracellular delivery is a central step in the research and development of engineered organisms. Existing technologies aimed at intracellular delivery of molecules rely on electrical fields, nanoparticles, or pore-forming chemicals. However, these methods suffer from numerous complications, including non-specific molecule delivery, modification or damage to the payload molecules, high cell death, low throughput, and/or difficult implementation. Due to their large size, complexes composed of biomolecules such as polypeptides, nucleic acids, carbohydrates, lipids, and/or small molecules cannot readily cross the cellular membrane. Thus, delivery of such complexes has been a challenge and there is an unmet need for intracellular delivery techniques that are highly effective at delivering complexes to a variety of cell types. In addition, techniques that allow for rapid, high throughput intracellular delivery of complexes can be applied more effectively to large scale clinical, manufacturing, and drug screening applications. References that describe methods of using channels to deliver compounds to cells include WO2013059343, WO2015023982, and PCT/US2015/058489.

All references cited herein, including patent applications and publications, are incorporated by reference in their entirety.

### BRIEF SUMMARY OF THE INVENTION

The invention provides methods for delivering a complex of two or more molecules into a cell, the method comprising passing a cell suspension through a constriction, wherein said constriction deforms the cell, thereby causing a perturbation of the cell such that the complex of molecules enters the cell, wherein said cell suspension is contacted with the complex of molecules. In some embodiments, formation of the complex of molecules is reversible. In some embodiments, at least two or more molecules of the complex associate by noncovalent interactions. In some embodiments, at least two molecules in the complex have a binding affinity in the complex ranging from about 1 µM to about 1 pM. In some embodiments, at least two molecules in the complex have a binding affinity in the complex ranging from about 1 µM to about 1 nM or from about 1 nM to about 1 pM. In some embodiments, the complex has a half-life in the cell suspension of about 1 minute to about 48 hours. In some embodiments, the complex has a half-life in the cell suspension of about 1 minute to about 20 minutes, about 20 minutes to about 40 minutes, about 40 minutes to about 1 hour, about 1 hour to about 2 hours, about 2 hours to about 6 hours, about 6 hours to about 12 hours, about 12 hours to about 24 hours, about 24 hours to about 36 hours, or about 36 hours to about 48 hours.

In some embodiment, the complex dissociates in the presence of a detergent. In some embodiments, the complex dissociates in the presence of a detergent at a concentration of about 0.1% (w/v) to about 10% (w/v). In some embodiments, the complex dissociates in the presence of a detergent at a concentration of about 0.1% (w/v) to about 1% (w/v), about 1% (w/v) to about 5% (w/v), or about 5% (w/v) to about 10% (w/v).

In some embodiments, the cell suspension is contacted with the complex of molecules at a temperature ranging from about 0 °C to about 40 °C. In some embodiments, the complex of molecules dissociates at a temperature greater than the temperature at which the cell suspension is contacted with the complex of molecules. In some embodiments, the complex of molecules dissociates at a temperature of about 50 °C to about 70 °C. In some embodiments, the complex of molecules dissociates at a temperature of about 50 °C to about 60 °C, or about 60 °C to about 70 °C.

In some embodiments, the cell suspension is contacted with the complex of molecules at an ionic strength ranging from about 50 mM to about 300 mM. In some embodiments, the complex of molecules dissociates at an ionic strength greater than the ionic strength at which the cell suspension is contacted with the complex of molecules. In some embodiments, the complex of molecules dissociates at an ionic strength of about 350 mM to about 1000 mM. In some embodiments, the complex of molecules dissociates at an ionic strength of about 350 mM to about 400 mM, about 400 mM to about 500 mM, about 500 mM to about 600 mM, about 700 mM to about 800 mM, about 800 mM to about 900 mM, or about 900 mM to about 1000 mM. In some embodiments, the complex of molecules dissociates at an ionic strength less than the ionic strength at which the cell suspension is contacted with the complex of molecules. In some embodiments, the complex of molecules dissociates at an ionic strength of about 0 mM to about 50 mM. In some embodiments, the complex of molecules dissociates at an ionic strength of about 0 mM to about 10 mM, about 10 mM to about 20 mM, about 20 mM to about 30 mM, about 30 mM to about 40 mM, or about 40 mM to about 50 mM.

In some embodiments, the cell suspension is contacted with the complex of molecules at an osmolarity ranging from about 100 mOsm/L to about 500 mOsm/L. In some embodiments, the complex of molecules dissociates at an osmolarity greater than the ionic strength at which the cell suspension is contacted with the complex of molecules. In some embodiments, the complex of molecules dissociates at an osmolarity of about 600 mOsm/L to about 1000 mOsm/L. In some embodiments, the complex of molecules dissociates at an osmolarity of about 600 mOsm/L to about 700 mOsm/L, about 700 mOsm/L to about 800 mOsm/L, about 800 mOsm/L to about 900 mOsm/L, or about 900 mOsm/L to about 1000 mOsm/L. In some embodiments, the complex of molecules dissociates at an osmolarity less than the osmolarity at which the cell suspension is contacted with the complex of molecules. In some embodiments, the complex of molecules dissociates at an osmolarity of about 0 mOsm/L to about 100 mOsm/L. In some embodiments, the complex of molecules dissociates at an osmolarity of about 0 mOsm/L to about 20 mOsm/L, about 20 mOsm/L to about 20 mOsm/L, about 20 mOsm/L to about 40 mOsm/L, about 40 mOsm/L to about 60 mOsm/L, about 60 mOsm/L to about 80 mOsm/L, or about 80 mOsm/L to about 100 mOsm/L.

In some embodiments, the cell suspension is contacted with the complex of molecules at a pH ranging from about 5.5 to about 8.5. In some embodiments, the complex of molecules dissociates at a pH greater or lower than the pH at which the cell suspension is contacted with the complex of molecules. In some embodiments, the complex of molecules dissociates at a pH of about 4.0 to about 5.5 or at a pH of about 8.5 to about 10. In some embodiments, the complex of molecules dissociates at a pH of about 4.0 to about 4.5, about 4.5 to about 5.0, about 5.0 to about 5.5, about 8.5 to about 9.0, about 9.0 to about 9.5, or about 9.5 to about 10.0.

In some embodiments, the shear force as the cell passes through the constriction ranges from about 1 kPa to about 10 kPa. In some embodiments, the complex dissociates at a shear force of about 10 kPa to about 100 kPa. In some embodiments, the complex dissociates at a shear force of about 10 kPa to about 25 kPa, about 25 kPa to about 50 kPa, about 50 kPa to about 75 kPa, or about 75 kPa to about 100 kPa.

In some embodiments, the complex of molecules comprises a) one or more polypeptides, b) one or more nucleic acids, c) one or more lipids, d) one or more carbohydrates, e) one or more small molecules, f) one or more metal-containing compounds, g) one or more polypeptides and one or more nucleic acids, h) one or more polypeptides and one or more lipids, i) one or more polypeptides and one or more carbohydrates, j) one or more polypeptides and one or more small molecules, k) one or more polypeptides and one or more metal-containing compounds, 1) one or more nucleic acids and one or more lipids, m) one or more nucleic acids and one or more carbohydrates, n) one or more nucleic acids and one or more small molecules, o) one or more nucleic acids and one or more metal-containing compounds, p) one or more lipids and one or more carbohydrates, q) one or more lipids and one or more small molecules, r) one or more lipids and one or more metal-containing compounds, s) one or more carbohydrates and one or more small molecules, t) one or more carbohydrates and one or more metal-containing compounds, u) one or more small molecules and one or more metal-containing compounds, v) one or more polypeptides, one or more nucleic acids and one or more lipids, w) one or more polypeptides, one or more nucleic acids and one or more carbohydrate, x) one or more polypeptides, one or more nucleic acids and one or more small molecules, y) one or more polypeptides, one or more nucleic acids and one or more metal-containing compounds, z) one or more polypeptides, one or more lipids and one or more carbohydrates, aa) one or more polypeptides, one or more lipids and one or more small molecules, ab) one or more polypeptides, one or more lipids and one or more metal-containing compounds, ac) one or more polypeptides, one or more carbohydrates and one or more small molecules, ad) one or more polypeptides, one or more carbohydrates and one or more metal-containing compounds, ae) one or more polypeptides, one or more small molecules and one or more metal-containing compounds, af) one or more nucleic acids, one or more lipids, and one or more carbohydrates, ag) one or more nucleic acids, one or more lipids, and one or more small molecules, ah) one or more nucleic acids, one or more lipids, and one or more metal-containing compounds, ai) one or more nucleic acids, one or more carbohydrates, and one or more small molecules, aj) one or more nucleic acids, one or more carbohydrates, and one or more metal-containing compounds, ak) one or more nucleic acids, one or more small molecules, and one or more metal-containing compounds, al) one or more lipids, one or more carbohydrates and one or more small molecules, am) one or more lipids, one or more carbohydrates and one or more metal-containing compounds, an) one or more lipids, one or more small molecules and one or more metal-containing compounds, ao) one or more carbohydrates, one or more small molecules and one or more metal-containing compounds, ap) one or more polypeptides, one or more nucleic acids, one or more lipids, and one or more carbohydrates, aq) one or more polypeptides, one or more nucleic acids, one or more lipids, and one or more small molecules, ar) one or more polypeptides, one or more nucleic acids, one or more lipids, and one or more metal-containing compounds, as) one or more polypeptides, one or more nucleic acids, one or more carbohydrates, and one or more small molecules, at) one or more polypeptides, one or more nucleic acids, one or more carbohydrates, and one or more metal-containing compounds, au) one or more polypeptides, one or more nucleic acids, one or more small molecules, and one or more metal-containing compounds, av) one or more polypeptides, one or more lipids, one or more carbohydrates, and one or more small molecules, aw) one or more polypeptides, one or more lipids, one or more carbohydrates, and one or more metal-containing compounds, ax) one or more polypeptides, one or more lipids, one or more small molecules, and one or more metal-containing compounds, ay) one or more polypeptides, one or more carbohydrates, one or more small molecules, and one or more metal-containing compounds, az) one or more nucleic acids, one or more lipids, one or more carbohydrates, and one or more small molecules, ba) one or more nucleic acids, one or more lipids, one or more carbohydrates, and one or more metal-containing compounds, bb) one or more nucleic acids, one or more lipids, one or more small molecules, and one or more metal-containing compounds, be) one or more nucleic acids, one or more carbohydrates, one or more small molecules, and one or more metal-containing compounds, bd) one or more lipids, one or more carbohydrates, one or more small molecules, and one or more metal-containing compounds, be) one or more polypeptides, one or more nucleic acids, one or more lipids, one or more carbohydrates, and one or more small molecules, bf) one or more polypeptides, one or more nucleic acids, one or more lipids, one or more carbohydrates, and one or more metal-containing compounds, bg) one or more polypeptides, one or more nucleic acids, one or more lipids, one or more small molecules, and one or more metal-containing compounds, bh) one or more polypeptides, one or more nucleic acids, one or more carbohydrates, one or more small molecules, and one or more metal-containing compounds, bi) one or more polypeptides, one or more lipids, one or more carbohydrates, one or more small molecules, and one or more metal-containing compounds, bj) one or more nucleic acids, one or more lipids, one or more carbohydrates, one or more small molecules, and one or more metal-containing compounds, or bk) one or more polypeptides, one or more nucleic acids, one or more lipids, one or more carbohydrates, one or more small molecules, and one or more metal-containing compounds. In some embodiments, the complex comprises an antibody. In some embodiments, the complex comprises one or more transcription factors. In some embodiments, the complex comprises a ribosome and an mRNA. In some embodiments, the complex comprises a proteasome, a holoenzyme, an RNA polymerase, a DNA polymerase, a spliceosome, a vault cytoplasmic ribonucleoprotein, a small nuclear ribonucleic protein (snRNP), a telomerase, a nucleosome, a death signaling complex (DISC), a mammalian target of rapamycin complex 1 (mTORC1), a mammalian target of rapamycin complex 2 (mTORC2), or a class I phosphoinositide 3 kinase (Class I PI3K), RNA-induced silencing complex (RISC), histone-DNA complex, toll-like receptor (TLR)-agonist complex, transposase/transposon complex, tRNA ribosome complex, polypeptide-protease complex, or an enzyme-substrate complex.

In some embodiments, the cell suspension is contacted with the complex after the cell suspension passes through the constriction. In some embodiments, the cell suspension is contacted with the complex before the cell suspension passes through the constriction. In some embodiments, the cell suspension is contacted with the complex at the same time the cell suspension passes through the constriction. In some embodiments, the complex is formed prior to contact with the cell suspension. In some embodiments, the complex is formed about 1 minute, about 5 minutes, about 10 minutes, about 15 minutes, about 30 minutes, about 45 minutes, about 1 hour, about 2 hours, about 3 hours, or about 6 hours prior to contact with the cell suspension. In some embodiments, the complex is purified prior to contact with the cell suspension. In some embodiments, the complex is formed in the cell suspension. In some embodiments, one or more of the molecules of the complex are purified prior to contact with the cell suspension.

In some embodiments, the cell suspension comprises a mixed cell population. In some embodiments, the cell suspension comprises a purified cell population. In some embodiments, the cell suspension comprises prokaryotic or eukaryotic cells. In some embodiments, the cell suspension comprises bacterial cells, archael cells, yeast cells, fungal cells, algal cells, plant cells or animal cells. In some embodiments, the cell suspension comprises vertebrate cells. In some embodiments, the cell suspension comprises mammalian cells. In some embodiments, the cell suspension comprises human cells.

In some embodiments, the constriction is contained within a microfluidic channel. In some embodiments, the constriction is a pore or contained within a pore. In some embodiments, the pore is contained in a surface. In some embodiments, the surface is a filter. In some embodiments, the surface is a membrane. In some embodiments, the pore size is about 0.4 µm, about 1 µm, about 2 µm, about 3 µm, about 4 µm, about 5 µm, about 6 µm, about 7 µm, about 8 µm, about 9 µm, about 10 µm, about 11 µm, about 12 µm, about 13 µm, or about 14 µm. In some embodiments, the constriction size is a function of the cell diameter. In some embodiments, the constriction size is about 20%, about 30%, about 40%, about 50%, about 60%, about 70%, about 80%, about 90%, or about 99% of the cell diameter. In some embodiments, the constriction has a length of about 30 µm and a width of about 3 µm to about 8 µm (such as about any of 4, 5, 6, or 7 µm). In some embodiments, the constriction has a length of about 10 µm and a width of about 3 µm to about 8 µm (such as about any of 4, 5, 6, or 7 µm). In some embodiments, the method further comprises the step of contacting the cell suspension and complex with an electric field generated by at least one electrode.

In some embodiments, the invention provides a system for delivering a complex of two or more molecules into a cell, the system comprising a microfluidic channel comprising a constriction, a cell suspension comprising the cell, and the complex of two or more molecules; wherein the constriction is configured such that the cell can pass through the constriction wherein the constriction deforms the cell thereby causing a perturbation of the cell such that the complex of two or more molecules enters the cell. In other embodiments, the invention provides a system for delivering a complex of two or more molecules into a cell, the system comprising a surface with pores, a cell suspension comprising the cell, and the complex of two or more molecules; wherein the surface with pores is configured such that the cell can pass through the pore wherein the pore deforms the cell thereby causing a perturbation of the cell such that the complex of two or more molecules enters the cell. In some embodiments, the surface is a filter or a membrane. In some embodiments, the system further comprises at least one electrode to generate an electric field. In some embodiments, formation of the complex of molecules is reversible. In some embodiments, at least two or more molecules of the complex associate by noncovalent interactions. In some embodiments, the system is used to deliver a complex comprising two or more molecules into a cell by any of the methods described herein.

In some embodiments, the invention provides cell comprising a complex of two or more molecules, wherein the complex of two or more molecules was delivered into the cell by any of the methods described herein.

### BRIEF DESCRIPTION OF THE DRAWINGS

FIG. 1A shows representative flow cytometry histogram plots demonstrating delivery of 3kDa Cascade Blue dextran and fluorescently labeled anti-CAS9 antibody to HEK293 cells by passage through a microfluidic chip with a 7 µm width constriction. RNP + Ab: anti-CAS9 antibody/CAS9/gRNA complex; RNP: CAS9/gRNA, Antibody: anti-CAS9 antibody; Endocytosis (no device): anti-CAS9 antibody/CAS9/gRNA complex without constriction.
FIG. 1B shows quantification of the percentage of cells from FIG. 1A positive for 3kDa Cascade Blue dextran and fluorescently labeled anti-CAS9 antibody. White bars: 3kDa Cascade Blue dextran; Black bars: fluorescently labeled anti-CAS9 antibody.
FIG. 2A shows representative FACS contour plots demonstrating B2M knockdown at day 6 following constriction-mediated delivery of functional complexes containing CAS9/gRNA RNPs. RNP + Antibody: anti-CAS9 antibody/CAS9/gRNA complex; RNP: CAS9/gRNA, Antibody: anti-CAS9 antibody; Endocytosis: anti-CAS9 antibody/CAS9/gRNA complex without constriction.
FIG. 2B shows quantification of the percentage of cells from FIG. 2A exhibiting knockdown of B2M.

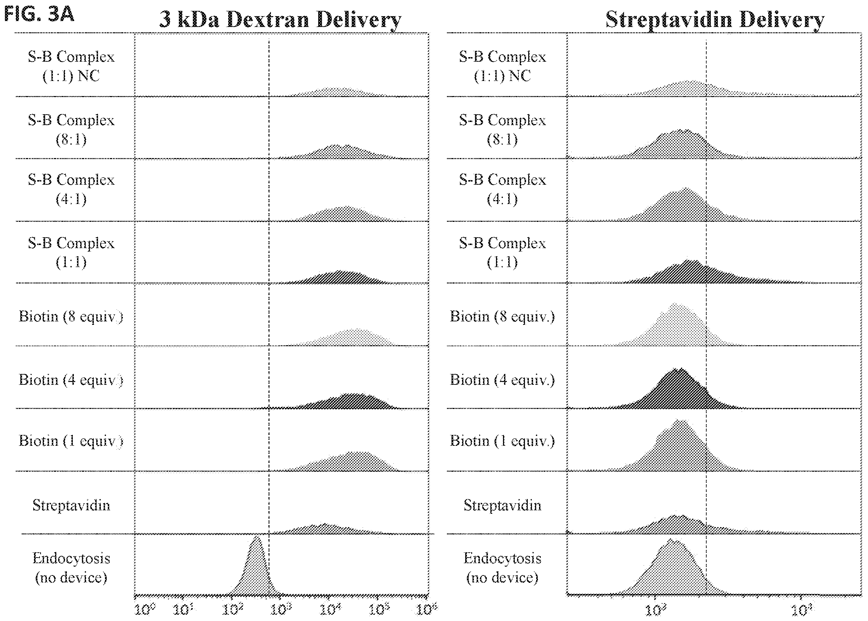

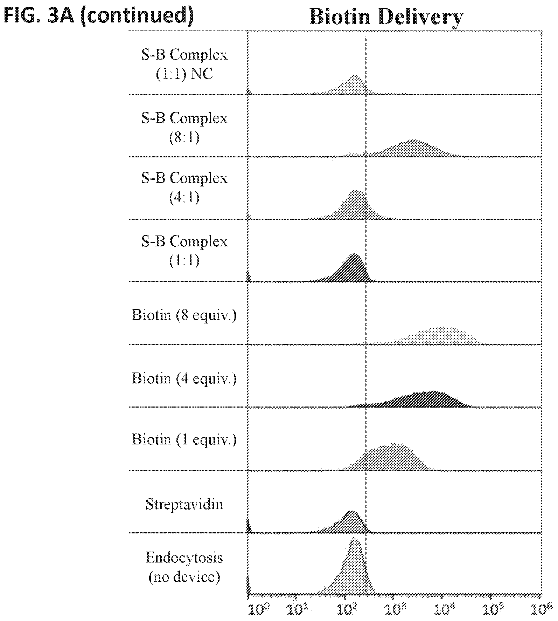

FIG. 3A shows representative flow cytometry histogram plots demonstrating delivery of AlexaFluor 680-labeled 3kDa dextran, Pacific Blue-labeled streptavidin, and FITC-labeled biotin to HeLa cells by passage through a microfluidic chip with a 7 µm width constriction. S-B Complex (1:1) NC: biotin:streptavidin at 1:1 molar ratio without preincubation prior to constriction-mediated delivery; S-B Complex (8:1): biotin:streptavidin complex at 8:1 molar ratio; S-B Complex (4:1): biotin:streptavidin complex at 4:1 molar ratio; S-B Complex (1:1): biotin:streptavidin complex at 1:1 molar ratio; Biotin (8 equiv.): biotin alone at 16 µM; Biotin (4 equiv.): biotin alone at 8 µM; Biotin (1 equiv.): biotin alone at 2 µM; Streptavidin: streptavidin alone at 2 µM; Endocytosis (no device): biotin: streptavidin complex at 1:1 molar ratio without constriction.
FIG. 3B shows quantification of the percentage of cells from FIG. 3A positive for 3kDa AlexaFluor 680 dextran, Pacific Blue-labeled streptavidin, or FITC-labeled biotin.
FIG. 3C shows representative FACS contour plots for the cells from FIG. 3A.
FIG. 4A shows representative flow cytometry histogram plots demonstrating delivery of 3kDa AlexaFluor 680 dextran and Pacific Blue-labeled streptavidin to HeLa cells by passage through a microfluidic chip with a 7 µm width constriction. SA + B-Phall: complex containing streptavidin and phalloidin-conjugated biotin; SA: streptavidin alone; B-Phall: phalloidin-conjugated biotin alone; Endocytosis (no device): complex containing streptavidin and phalloidin-conjugated biotin without constriction.
FIG. 4B shows quantification of the percentage of cells from FIG. 4A positive for 3kDa AlexaFluor 680 dextran and Pacific Blue-labeled streptavidin. White bars: 3kDa AlexaFluor 680 dextran; Grey bars: Pacific Blue-labeled streptavidin.
FIG. 5 shows representative FACS plots for the cells from FIG. 4A demonstrating correlation between 3kDa AlexaFluor 680 dextran delivery and Pacific Blue-labeled streptavidin delivery.
FIGS. 6A-6D show the results for delivery of 3kDa-Cascade Blue dextran in combination with HPV16 E7 SLP alone (Uncomplexed E7 SLP) or a complex containing HPV16 E7 SLP and mouse serum albumin (Complexed E7 SLP/MSA) to T cells isolated from C57BL/6 mice by passage through a microfluidic chip with a 3 µm width constriction (FIGS. 6A and 6C) and resultant cell viability (FIGS. 6B and 6D). SQZ: with constriction; Endo: without constriction.
FIG. 7 shows representative FACS histogram plots for the cells from FIGS. 6A and 6C.
FIG. 8 shows the endogenous CD8 T-cell response as measured by tetramer staining six days after the T cells from FIGS. 6A and 6C were introduced back into the mice. NC: negative control T cells with no antigen; Endo: HPV16 E7 SLP alone without constriction; Endo + MSA: complex containing HPV16 E7 SLP and MSA without constriction; SQZ: HPV16 E7 SLP alone with constriction; SQZ + MSA: complex containing HPV16 E7 SLP and MSA with constriction.

### DETAILED DESCRIPTION

The invention provides methods for delivering a complex of two or more molecules into a cell, the method comprising passing a cell suspension through a constriction, wherein said constriction deforms the cell, thereby causing a perturbation of the cell such that the complex of molecules enters the cell, wherein said cell suspension is contacted with the complex of molecules. In some embodiments, the complex is a transient, reversible complex. In some embodiments, the complex comprises component compounds held together by non-covalent interactions. In some embodiments, the constriction is contained within a microfluidic channel. In some embodiments, the constriction is a pore or contained within a pore. In some embodiments, the pore is contained in a surface. In some embodiments, the surface is a filter. In some embodiments, the surface is a membrane.

### I. GENERAL TECHNIQUES

The techniques and procedures described or referenced herein are generally well understood and commonly employed using conventional methodology by those skilled in the art, such as, for example, the widely utilized methodologies described in Molecular Cloning: A Laboratory Manual (Sambrook et al., 4th ed., Cold Spring Harbor Laboratory Press, Cold Spring Harbor, N.Y., 2012); Current Protocols in Molecular Biology (F.M. Ausubel, et al. eds., 2003); the series Methods in Enzymology (Academic Press, Inc.); PCR 2: A Practical Approach (M.J. MacPherson, B.D. Hames and G.R. Taylor eds., 1995); Antibodies, A Laboratory Manual (Harlow and Lane, eds., 1988); Culture of Animal Cells: A Manual of Basic Technique and Specialized Applications (R.I. Freshney, 6th ed., J. Wiley and Sons, 2010); Oligonucleotide Synthesis (M.J. Gait, ed., 1984); Methods in Molecular Biology, Humana Press; Cell Biology: A Laboratory Notebook (J.E. Cellis, ed., Academic Press, 1998); Introduction to Cell and Tissue Culture (J.P. Mather and P.E. Roberts, Plenum Press, 1998); Cell and Tissue Culture: Laboratory Procedures (A. Doyle, J.B. Griffiths, and D.G. Newell, eds., J. Wiley and Sons, 1993-8); Handbook of Experimental Immunology (D.M. Weir and C.C. Blackwell, eds., 1996); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Ausubel et al., eds., J. Wiley and Sons, 2002); Immunobiology (C.A. Janeway et al., 2004); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane, Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 2011).

### II. DEFINITIONS

For purposes of interpreting this specification, the following definitions will apply and whenever appropriate, terms used in the singular will also include the plural and vice versa. In the event that any definition set forth below conflicts with any document incorporated herein by reference, the definition set forth shall control.

As used herein, the singular form "a", "an", and "the" includes plural references unless indicated otherwise.

It is understood that aspects and embodiments of the disclosure described herein include "comprising," "consisting," and "consisting essentially of" aspects and embodiments.

For all compositions described herein, and all methods using a composition described herein, the compositions can either comprise the listed components or steps, or can "consist essentially of" the listed components or steps. When a composition is described as "consisting essentially of" the listed components, the composition contains the components listed, and may contain other components which do not substantially affect the methods disclosed, but do not contain any other components which substantially affect the methods disclosed other than those components expressly listed; or, if the composition does contain extra components other than those listed which substantially affect the methods disclosed, the composition does not contain a sufficient concentration or amount of the extra components to substantially affect the methods disclosed. When a method is described as "consisting essentially of" the listed steps, the method contains the steps listed, and may contain other steps that do not substantially affect the methods disclosed, but the method does not contain any other steps which substantially affect the methods disclosed other than those steps expressly listed. As a non-limiting specific example, when a composition is described as 'consisting essentially of' a component, the composition may additionally contain any amount of pharmaceutically acceptable carriers, vehicles, or diluents and other such components which do not substantially affect the methods disclosed.

The term "about" as used herein refers to the usual error range for the respective value readily known to the skilled person in this technical field. Reference to "about" a value or parameter herein includes (and describes) embodiments that are directed to that value or parameter per se.

The term "complex" as used herein refers to a chemical association of two or more molecules, species or compounds by non-covalent bonds. The two or more molecules are joined, for example without limitation, by weak electrostatic bonds, hydrophobic interactions, van der waals interactions, *etc.* In some examples, the complex may be composed of a number of different compounds, including without limitation, polypeptides, nucleic acids, carbohydrates, lipids, small molecules, and/or metal-containing compounds.

The terms "polypeptide complex" and "protein complex" refer to a composite unit arising from the specific binding of a polypeptide with a binding partner, wherein said binding partner can be one or more polypeptides, one or more nucleic acids, or a combination of one or more polypeptides and one or more nucleic acids, and the like, to form said polypeptide complex. Polypeptide complexes may be polypeptide-polypeptide complexes, polypeptide-nucleic acid complexes, and the like. In certain embodiments, a polypeptide complex may comprise polypeptide-polypeptide interactions, e.g. interactions between different polypeptides, or dimers, trimers, tetramers or higher oligomers of the same polypeptide. Interactions between subunits of polypeptide complexes (e.g., in polypeptide-polypeptide complexes or polypeptide-nucleic acid complexes that comprise more than one polypeptide) or between polypeptides and nucleic acids (e.g., in polypeptide-nucleic acid complexes) are usually non-binding interactions, such as those interactions caused by hydrogen bridges, pi electron systems such as (optionally conjugated) C--C double bonds or aromatic rings, e.g. phenyl, and heteroaromatic rings, e.g. pyrrole, imidazole, indole, pyrimidine or purine rings, and interactions between metal atoms and oxygen, nitrogen or sulfur atoms, but may also be weak, and in particular reversible, covalent binding interactions, e.g. sulfur-sulfur bridges.

A "polypeptide-polypeptide" or "protein-protein complex" refers to a composite unit that is a combination of two or more polypeptides formed by interaction between the polypeptides. Typically but not necessarily, a "polypeptide complex" is formed by the binding of two or more polypeptides together through specific non-covalent binding affinities.

The term "constriction" as used herein refers to a narrowed passageway. In some examples, the constriction is contained within a microfluidic channel. In other examples, the constriction is a pore or contained within a pore. In some examples where the constriction is a pore, the pore is contained in a surface.

The term "pore" as used herein refers to an opening, including without limitation, a hole, tear, cavity, aperture, break, gap, or perforation within a material. In some examples, (where indicated) the term refers to a pore within a surface of the present disclosure. In other examples, (where indicated) a pore can refer to a pore in a cell wall and/or cell membrane.

The term "membrane" as used herein refers to a selective barrier or sheet containing pores. The term includes a pliable sheetlike structure that acts as a boundary or lining. In some examples, the term refers to a surface or filter containing pores. This term is distinct from the term "cell membrane".

The term "filter" as used herein refers to a porous article that allows selective passage through the pores. In some examples the term refers to a surface or membrane containing pores.

The term "heterogeneous" as used herein refers to something which is mixed or not uniform in structure or composition. In some examples the term refers to pores having varied sizes, shapes or distributions within a given surface.

The term "homogeneous" as used herein refers to something which is consistent or uniform in structure or composition throughout. In some examples the term refers to pores having consistent sizes, shapes, or distribution within a given surface.

The term "heterologous" as used herein refers to a molecule which is derived from a different organism. In some examples the term refers to a nucleic acid or polypeptide which is not normally found or expressed within the given organism.

The term "homologous" as used herein refers to a molecule which is derived from the same organism. In some examples the term refers to a nucleic acid or polypeptide which is normally found or expressed within the given organism.

The term "polynucleotide" or "nucleic acid" as used herein refers to a polymeric form of nucleotides of any length, either ribonucleotides or deoxyribonucleotides. Thus, this term includes, but is not limited to, single-, double- or multi-stranded DNA or RNA, genomic DNA, cDNA, DNA-RNA hybrids, or a polymer comprising purine and pyrimidine bases, or other natural, chemically or biochemically modified, non-natural, or derivatized nucleotide bases. The backbone of the polynucleotide can comprise sugars and phosphate groups (as may typically be found in RNA or DNA), or modified or substituted sugar or phosphate groups. Alternatively, the backbone of the polynucleotide can comprise a polymer of synthetic subunits such as phosphoramidates and thus can be an oligodeoxynucleoside phosphoramidate (P-NH2) or a mixed phosphoramidate- phosphodiester oligomer. In addition, a double-stranded polynucleotide can be obtained from the single stranded polynucleotide product of chemical synthesis either by synthesizing the complementary strand and annealing the strands under appropriate conditions, or by synthesizing the complementary strand de novo using a DNA polymerase with an appropriate primer.

The terms "polypeptide" and "protein" are used interchangeably to refer to a polymer of amino acid residues, and are not limited to a minimum length. Such polymers of amino acid residues may contain natural or non-natural amino acid residues, and include, but are not limited to, peptides, oligopeptides, dimers, trimers, and multimers of amino acid residues. Both full-length proteins and fragments thereof are encompassed by the definition. The terms also include post-expression modifications of the polypeptide, for example, glycosylation, sialylation, acetylation, phosphorylation, and the like. Furthermore, for purposes of the present invention, a "polypeptide" refers to a protein which includes modifications, such as deletions, additions, and substitutions (generally conservative in nature), to the native sequence, as long as the protein maintains the desired activity. These modifications may be deliberate, as through site-directed mutagenesis, or may be accidental, such as through mutations of hosts which produce the proteins or errors due to PCR amplification.

For any of the structural and functional characteristics described herein, methods of determining these characteristics are known in the art.

### III. COMPLEXES TO DELIVER

In certain aspects, the present disclosure relates to methods for delivering a complex into a cell. In some embodiments, the complex is a single complex. In some embodiments, the complex is a mixture of complexes. In some embodiments, a complex or mixture of complexes is delivered to a cell to produce a desired effect.

In some embodiments, the complex is a transient, reversible complex. In some embodiments, the complex comprises component compounds held together by non-covalent interactions. In some embodiments, the complex is delivered to cells under conditions whereby the complex remains intact such that the complex performs a desired function once inside the cell. In some embodiments, the non-covalent interactions allow the complex to dissociate once delivered to the cell, allowing for the separate complex components to perform a desired function once inside the cell.

One or more parameters may be used to characterize the complex of two or more molecules that are delivered to cells by the methods of the invention as outlined below.

In some embodiments, at least two molecules in the complex have a binding affinity in the complex ranging from about 1 µM to about 1 pM. In some embodiments, at least two molecules in the complex have a binding affinity in the complex ranging from any one of about 1 µM to about 10 µM, about 10 µM to about 100 µM, about 100 µM to about 1 nM, about 1 nM to about 10 nM, about 10 nM to about 100 nM, or from about 100 nM to about 1 pM.

In some embodiments, the complex of the invention has a half-life under physiologic conditions of about 1 minute to greater than about 48 hours. In some embodiments, the complex has a half-life in the cell suspension of about 1 minute to greater than about 48 hours. In some embodiments, the complex has a half-life in the cell suspension of greater than any of about 1 minute, 2 minutes, 3 minutes, 4 minutes, 5 minutes, 6 minutes, 7 minutes, 8 minutes, 9 minutes, 10 minutes, 20 minutes, 30 minutes, 40 minutes, 50 minutes or 60 minutes. In some embodiments, the complex has a half-life in the cell suspension of greater than any of about 1 hour, 2 hours, 3 hours, 4 hours, 5 hours, 6 hours, 7 hours, 8 hours, 9 hours, 10 hours, 11 hours, 12 hours, 18 hours, 24 hours or 48 hours. In some embodiments, the complex has a half-life in the cell suspension of about 1 minute to about 20 minutes, about 20 minutes to about 40 minutes, about 40 minutes to about 1 hour, about 1 hour to about 2 hours, about 2 hours to about 6 hours, about 6 hours to about 12 hours, about 12 hours to about 24 hours, about 24 hours to about 36 hours, or about 36 hours to about 48 hours. In some embodiments, the complex has a half-life in the cell of about 1 minute to greater than about 48 hours. In some embodiments, the complex has a half-life in the cell suspension of greater than any of about 1 minute, 2 minutes, 3 minutes, 4 minutes, 5 minutes, 6 minutes, 7 minutes, 8 minutes, 9 minutes, 10 minutes, 20 minutes, 30 minutes, 40 minutes, 50 minutes or 60 minutes. In some embodiments, the complex has a half-life in the cell of greater than any of about 1 hour, 2 hours, 3 hours, 4 hours, 5 hours, 6 hours, 7 hours, 8 hours, 9 hours, 10 hours, 11 hours, 12 hours, 18 hours, 24 hours or 48 hours. In some embodiments, the complex has a half-life in the cell of about 1 minute to about 20 minutes, about 20 minutes to about 40 minutes, about 40 minutes to about 1 hour, about 1 hour to about 2 hours, about 2 hours to about 6 hours, about 6 hours to about 12 hours, about 12 hours to about 24 hours, about 24 hours to about 36 hours, or about 36 hours to about 48 hours.

In some embodiments of the invention, the complex of two or more molecules is characterized by association of the molecules of the complex or by the parameters that result in dissociation of the complex. In some embodiments, a dissociated complex is a complex wherein the association of two or more molecules in the complex is disrupted. In some embodiments, a dissociated complex is one wherein greater than about any of 10%, 20%, 30%, 40%, 50%, 60%, 70%, 80%, 90%, or 100% of the complexes comprise a disruption in the association of two or more molecules in the complex.

In some embodiments, the complex dissociates in the presence of a detergent, a surfactant, an organic solvent, a chaotropic agent. Examples of detergents and surfactants include but are not limited to polysorbates, sodium dodecyl sulfate (SDS), CHAPS, poloxamers, Triton X-100, NP-40. Examples of organic solvents include but are not limited to ethanol, propanol, butanol, acetic acid, formic acid, dichloromethane, ethyl acetate, acetonitrile, dimethylformamide, acetonitrile, and dimethyl sulfoxide. Examples of chaotropic agents include but are not limited to butanol, ethanol, guanidinium chloride, lithium perchlorate, lithium acetate, magnesium chloride, phenol, propanol, SDS, thiourea and urea. In some embodiments, the complex of the invention dissociates in the presence of a detergent at a concentration of about 0.1% to about 10%. In some embodiments, the complex of the invention dissociates in the presence of a detergent at a concentration of greater any of about 0.1%, 0.2%, 0.3%, 0.4%, 0.5%, 0.6%, 0.7%, 0.8%, 0.9%, 1.0%, 2.0%, 3.0%, 4.0%, 5.0%, 6.0%, 7.0%, 8.0%, 9.0%, or 10.0%. In some embodiments, the complex dissociates in the presence of a detergent at a concentration of about 0.1% to about 0.2%, about 0.2% to about 0.3%, about 0.3% to about 0.4%, about 0.4% to about 0.5%, about 0.5% to about 0.6%, about 0.6% to about 0.7%, about 0.7% to about 0.8%, about 0.8% to about 0.9%, about 0.9% to about 1.0%, about 1.0% to about 2.0%, about 2.0% to about 3.0%, about 3.0% to about 4.0%, about 4.0% to about 5.0%, about 5.0% to about 6.0%, about 6.0% to about 7.0%, about 07.0% to about 8.0%, about 8.0% to about 9.0%, or about 9.0% to about 10.0%. In some embodiments, a detergent or surfactant is added to the cell suspension; for example, to prevent clogging of a microchannel or pore. In some embodiments, the detergent or surfactant is added to the cell suspension at a concentration that does not result in substantial dissociation of the complex of molecules to be delivered to the cell. In some embodiments, the detergent or surfactant is added to the cell suspension at a concentration that results in less than about 10%, 20%, 30%, 40%, 50%, 60%, 70%, 80%, or 90% dissociation of the complex of molecules to be delivered to the cell.

In some embodiments, the complex of two or more molecules dissociates at elevated temperature. In some embodiments, the complex of molecules dissociates in the cell suspension at a temperature greater than the temperature at which the cell suspension is contacted with the complex of molecules. In some embodiments, the cell suspension is contacted with the complex of two or more molecules at a temperature less than the temperature at which the complex dissociates in the cell suspension. In some embodiments, the cell suspension is contacted with the complex of two or more molecules at a temperature that is at least about 10%, 20%, 30%, 40%, 50%, 60%, 70%, 80%, or 90% less than the temperature at which the complex dissociates in the cell suspension. In some embodiments, the cell suspension is contacted with the complex of molecules at a temperature ranging from about 0 °C to about 40 °C. In some embodiments, the complex of molecules dissociates at a temperature of about 50 °C to about 70 °C. In some embodiments, the complex of molecules dissociates at a temperature of about 50 °C to about 60 °C, or about 60 °C to about 70 °C. In some embodiments, the cell suspension is contacted with the complex of two or more molecules at a temperature that does not result in substantial dissociation of the complex of molecules to be delivered to the cell. In some embodiments, the cell suspension is contacted with the complex of two or more molecules at a temperature that results in less than about 10%, 20%, 30%, 40%, 50%, 60%, 70%, 80%, or 90% dissociation of the complex of molecules to be delivered to the cell.

In some embodiments, the complex of two or more molecules dissociates in the cell suspension at elevated ionic strength. In some embodiments, the complex of molecules dissociates in the cell suspension at an ionic strength greater than the ionic strength at which the cell suspension is contacted with the complex of molecules. In some embodiments, the cell suspension is contacted with the complex of two or more molecules at an ionic strength less than the ionic strength at which the complex dissociates in the cell suspension. In some embodiments, the cell suspension is contacted with the complex of two or more molecules at an ionic strength that is at least about 10%, 20%, 30%, 40%, 50%, 60%, 70%, 80%, or 90% less than the ionic strength at which the complex dissociates in the cell suspension. In some embodiments, the cell suspension is contacted with the complex of molecules at an ionic strength ranging from about 50 mM to about 300 mM. In some embodiments, the complex of molecules dissociates at an ionic strength of about 350 mM to about 1000 mM. In some embodiments, the complex of molecules dissociates at an ionic strength of about 350 mM to about 400 mM, about 400 mM to about 500 mM, about 500 mM to about 600 mM, about 700 mM to about 800 mM, about 800 mM to about 900 mM, or about 900 mM to about 1000 mM. In some embodiments, the cell suspension is contacted with the complex of two or more molecules at an ionic strength that does not result in substantial dissociation of the complex of molecules to be delivered to the cell. In some embodiments, the cell suspension is contacted with the complex of two or more molecules at an ionic strength that results in less than about 10%, 20%, 30%, 40%, 50%, 60%, 70%, 80%, or 90% dissociation of the complex of molecules to be delivered to the cell.

In some embodiments, the complex of two or more molecules dissociates in the cell suspension at decreased ionic strength. In some embodiments, the complex of molecules dissociates in the cell suspension at an ionic strength less than the ionic strength at which the cell suspension is contacted with the complex of molecules. In some embodiments, the cell suspension is contacted with the complex of two or more molecules at an ionic strength greater than the ionic strength at which the complex dissociates in the cell suspension. In some embodiments, the cell suspension is contacted with the complex of two or more molecules at an ionic strength that is at least about 10%, 20%, 30%, 40%, 50%, 60%, 70%, 80%, 90% or 100% greater than the ionic strength at which the complex dissociates in the cell suspension. In some embodiments, the cell suspension is contacted with the complex of molecules at an ionic strength ranging from about 50 mM to about 300 mM. In some embodiments, the complex of molecules dissociates at an ionic strength of about 0 mM to about 50 mM. In some embodiments, the complex of molecules dissociates at an ionic strength of about 0 mM to about 10 mM, about 10 mM to about 20 mM, about 20 mM to about 30 mM, about 30 mM to about 40 mM, or about 40 mM to about 50 mM. In some embodiments, the cell suspension is contacted with the complex of two or more molecules at an ionic strength that does not result in substantial dissociation of the complex of molecules to be delivered to the cell. In some embodiments, the cell suspension is contacted with the complex of two or more molecules at an ionic strength that results in less than about 10%, 20%, 30%, 40%, 50%, 60%, 70%, 80%, or 90% dissociation of the complex of molecules to be delivered to the cell.

In some embodiments, the complex of two or more molecules dissociates in the cell suspension at elevated osmolarity. In some embodiments, the complex of molecules dissociates in the cell suspension at an osmolarity greater than the osmolarity at which the cell suspension is contacted with the complex of molecules. In some embodiments, the cell suspension is contacted with the complex of two or more molecules at an osmolarity less than the osmolarity at which the complex dissociates in the cell suspension. In some embodiments, the cell suspension is contacted with the complex of two or more molecules at an osmolarity that is at least about 10%, 20%, 30%, 40%, 50%, 60%, 70%, 80%, or 90% less than the osmolarity at which the complex dissociates in the cell suspension. In some embodiments, the cell suspension is contacted with the complex of molecules at an osmolarity ranging from about 100 mOsm/L to about 500 mOsm/L. In some embodiments, the complex of molecules dissociates at an osmolarity of about 600 mOsm/L to about 1000 mOsm/L. In some embodiments, the complex of molecules dissociates at an osmolarity of about 600 mOsm/L to about 700 mOsm/L, about 700 mOsm/L to about 800 mOsm/L, about 800 mOsm/L to about 900 mOsm/L, or about 900 mOsm/L to about 1000 mOsm/L. In some embodiments, the cell suspension is contacted with the complex of two or more molecules at an osmolarity that does not result in substantial dissociation of the complex of molecules to be delivered to the cell. In some embodiments, the cell suspension is contacted with the complex of two or more molecules at an osmolarity that results in less than about 10%, 20%, 30%, 40%, 50%, 60%, 70%, 80%, or 90% dissociation of the complex of molecules to be delivered to the cell.

In some embodiments, the complex of two or more molecules dissociates in the cell suspension at decreased osmolarity. In some embodiments, the complex of molecules dissociates in the cell suspension at an osmolarity less than the osmolarity at which the cell suspension is contacted with the complex of molecules. In some embodiments, the cell suspension is contacted with the complex of two or more molecules at an osmolarity greater than the osmolarity at which the complex dissociates in the cell suspension. In some embodiments, the cell suspension is contacted with the complex of two or more molecules at an osmolarity that is at least about 10%, 20%, 30%, 40%, 50%, 60%, 70%, 80%, 90% or 100% greater than the osmolarity at which the complex dissociates in the cell suspension. In some embodiments, the cell suspension is contacted with the complex of molecules at an osmolarity ranging from about 100 mOsm/L to about 500 mOsm/L. In some embodiments, the complex of molecules dissociates at an osmolarity of about 0 mOsm/L to about 100 mOsm/L. In some embodiments, the complex of molecules dissociates at an osmolarity of about 0 mOsm/L to about 20 mOsm/L, about 20 mOsm/L to about 40 mOsm/L, about 40 mOsm/L to about 60 mOsm/L, about 60 mOsm/L to about 80 mOsm/L, or about 80 mOsm/L to about 100 mOsm/L. In some embodiments, the cell suspension is contacted with the complex of two or more molecules at an osmolarity that does not result in substantial dissociation of the complex of molecules to be delivered to the cell. In some embodiments, the cell suspension is contacted with the complex of two or more molecules at an osmolarity that results in less than about 10%, 20%, 30%, 40%, 50%, 60%, 70%, 80%, or 90% dissociation of the complex of molecules to be delivered to the cell.

In some embodiments, the complex of two or more molecules dissociates at elevated pH. In some embodiments, the complex of molecules dissociates in the cell suspension at a pH greater than the pH at which the cell suspension is contacted with the complex of molecules. In some embodiments, the cell suspension is contacted with the complex of two or more molecules at a pH less than the pH at which the complex dissociates in the cell suspension. In some embodiments, the cell suspension is contacted with the complex of two or more molecules at a pH that is at least about 10%, 20%, 30%, 40%, 50%, 60%, 70%, 80%, or 90% less than the pH at which the complex dissociates in the cell suspension. In some embodiments, the cell suspension is contacted with the complex of molecules at a pH ranging from about 5.5 to about 8.5. In some embodiments, the complex of molecules dissociates at a pH of about 8.5 to about 10. In some embodiments, the complex of molecules dissociates at a pH of about 8.5 to about 9.0, about 9.0 to about 9.5, about 9.5 to about 10.0, about 10.00 to about 11.0, or about 11.0 to about 12.0. In some embodiments, the cell suspension is contacted with the complex of two or more molecules at a pH that does not result in substantial dissociation of the complex of molecules to be delivered to the cell. In some embodiments, the cell suspension is contacted with the complex of two or more molecules at a pH that results in less than about 10%, 20%, 30%, 40%, 50%, 60%, 70%, 80%, or 90% dissociation of the complex of molecules to be delivered to the cell.

In some embodiments, the complex of two or more molecules dissociates at lowered pH. In some embodiments, the complex of molecules dissociates in the cell suspension at a pH less than the pH at which the cell suspension is contacted with the complex of molecules. In some embodiments, the cell suspension is contacted with the complex of two or more molecules at a pH greater than the pH at which the complex dissociates in the cell suspension. In some embodiments, the cell suspension is contacted with the complex of two or more molecules at a pH that is at least about 10%, 20%, 30%, 40%, 50%, 60%, 70%, 80%, or 90% greater than the pH at which the complex dissociates in the cell suspension. In some embodiments, the cell suspension is contacted with the complex of molecules at a pH ranging from about 5.5 to about 8.5. In some embodiments, the complex of molecules dissociates at a pH of about 4.0 to about 5.5. In some embodiments, the complex of molecules dissociates at a pH of about 2.0 to about 3.0, about 3.5 to about 4.0, about 4.0 to about 4.5, about 4.5 to about 5.0, about 5.0 to about 5.5. In some embodiments, the cell suspension is contacted with the complex of two or more molecules at a pH that does not result in substantial dissociation of the complex of molecules to be delivered to the cell. In some embodiments, the cell suspension is contacted with the complex of two or more molecules at a pH that results in less than about 10%, 20%, 30%, 40%, 50%, 60%, 70%, 80%, or 90% dissociation of the complex of molecules to be delivered to the cell.

In some embodiments, the invention provides methods to deliver a complex of two or more molecules to a cell where a cell suspension containing the cell is passed through a constriction which perturbs the cell thus allowing the complex to enter the cell. As the cell suspension passes through the constriction, the cell suspension may be subject to shear forces. In some embodiments, the complex of two or more molecules dissociates under elevated shear force. In some embodiments, the complex of molecules dissociates in the cell suspension at a shear force greater than the shear force at which the cell suspension is contacted with the complex of molecules. In some embodiments, the cell suspension is contacted with the complex of two or more molecules at a shear force less than the shear force at which the complex dissociates in the cell suspension. In some embodiments, the cell suspension is contacted with the complex of two or more molecules at a shear force that is at least about 10%, 20%, 30%, 40%, 50%, 60%, 70%, 80%, or 90% less than the shear force at which the complex dissociates in the cell suspension. In some embodiments, the cell suspension is contacted with the complex of molecules at a shear force ranging from about 1 kPa to about 10 kPa. In some embodiments, the complex dissociates at a shear force of about 10 kPa to about 100 kPa. In some embodiments, the complex dissociates at a shear force of about 10 kPa to about 25 kPa, about 25 kPa to about 50 kPa, about 50 kPa to about 75 kPa, or about 75 kPa to about 100 kPa. In some embodiments, the cell suspension is contacted with the complex of two or more molecules at a shear force that does not result in substantial dissociation of the complex of molecules to be delivered to the cell. In some embodiments, the cell suspension is contacted with the complex of two or more molecules at a shear force that results in less than about 10%, 20%, 30%, 40%, 50%, 60%, 70%, 80%, or 90% dissociation of the complex of molecules to be delivered to the cell. In some embodiments, the cell suspension is contacted with the complex comprising two or more molecules after the cell suspension passes through the constriction to reduce the effects of shear force on the complex.

Methods to determine if a complex of two or more molecules are intact are known in the art, including but not limited to high performance liquid chromatography (particularly size exclusion chromatography), non-denaturing gel electrophoresis, immunoassays, mass spectroscopy, functional assays, resonance energy transfer-based assays (e.g., FRET or BRET) to determine if a complex with two fluorophores are in close proximity, histochemistry, and immunohistochemistry.

In some embodiments of the invention, the cell suspension is contacted with the complex after the cell suspension passes through the constriction. In some embodiments, the cell suspension is contacted with the complex before the cell suspension passes through the constriction. In some embodiments, the cell suspension is contacted with the complex at the same time the cell suspension passes through the constriction. In some embodiments, the complex is formed prior to contact with the cell suspension. In some embodiments, the complex is formed about 1 minute, about 5 minutes, about 10 minutes, about 15 minutes, about 30 minutes, about 45 minutes, about 1 hour, about 2 hours, about 3 hours, or about 6 hours prior to contact with the cell suspension. In some embodiments, the complex is formed greater than about 6 hours prior to contact with the cell suspension. In some embodiments, wherein the complex is formed in the cell suspension prior to entry into the cell.

The invention provides methods to deliver a complex of two or more molecules to a cell where a cell suspension containing the cell is passed through a constriction which perturbs the cell thus allowing the complex to enter the cell. In some embodiments, the complex of molecules comprises a) one or more polypeptides, b) one or more nucleic acids, c) one or more lipids, d) one or more carbohydrates, e) one or more small molecules, f) one or more metal-containing compounds, g) one or more polypeptides and one or more nucleic acids, h) one or more polypeptides and one or more lipids, i) one or more polypeptides and one or more carbohydrates, j) one or more polypeptides and one or more small molecules, k) one or more polypeptides and one or more metal-containing compounds, 1) one or more nucleic acids and one or more lipids, m) one or more nucleic acids and one or more carbohydrates, n) one or more nucleic acids and one or more small molecules, o) one or more nucleic acids and one or more metal-containing compounds, p) one or more lipids and one or more carbohydrates, q) one or more lipids and one or more small molecules, r) one or more lipids and one or more metal-containing compounds, s) one or more carbohydrates and one or more small molecules, t) one or more carbohydrates and one or more metal-containing compounds, u) one or more small molecules and one or more metal-containing compounds, v) one or more polypeptides, one or more nucleic acids and one or more lipids, w) one or more polypeptides, one or more nucleic acids and one or more carbohydrate, x) one or more polypeptides, one or more nucleic acids and one or more small molecules, y) one or more polypeptides, one or more nucleic acids and one or more metal-containing compounds, z) one or more polypeptides, one or more lipids and one or more carbohydrates, aa) one or more polypeptides, one or more lipids and one or more small molecules, ab) one or more polypeptides, one or more lipids and one or more metal-containing compounds, ac) one or more polypeptides, one or more carbohydrates and one or more small molecules, ad) one or more polypeptides, one or more carbohydrates and one or more metal-containing compounds, ae) one or more polypeptides, one or more small molecules and one or more metal-containing compounds, af) one or more nucleic acids, one or more lipids, and one or more carbohydrates, ag) one or more nucleic acids, one or more lipids, and one or more small molecules, ah) one or more nucleic acids, one or more lipids, and one or more metal-containing compounds, ai) one or more nucleic acids, one or more carbohydrates, and one or more small molecules, aj) one or more nucleic acids, one or more carbohydrates, and one or more metal-containing compounds, ak) one or more nucleic acids, one or more small molecules, and one or more metal-containing compounds, al) one or more lipids, one or more carbohydrates and one or more small molecules, am) one or more lipids, one or more carbohydrates and one or more metal-containing compounds, an) one or more lipids, one or more small molecules and one or more metal-containing compounds, ao) one or more carbohydrates, one or more small molecules and one or more metal-containing compounds, ap) one or more polypeptides, one or more nucleic acids, one or more lipids, and one or more carbohydrates, aq) one or more polypeptides, one or more nucleic acids, one or more lipids, and one or more small molecules, ar) one or more polypeptides, one or more nucleic acids, one or more lipids, and one or more metal-containing compounds, as) one or more polypeptides, one or more nucleic acids, one or more carbohydrates, and one or more small molecules, at) one or more polypeptides, one or more nucleic acids, one or more carbohydrates, and one or more metal-containing compounds, au) one or more polypeptides, one or more nucleic acids, one or more small molecules, and one or more metal-containing compounds, av) one or more polypeptides, one or more lipids, one or more carbohydrates, and one or more small molecules, aw) one or more polypeptides, one or more lipids, one or more carbohydrates, and one or more metal-containing compounds, ax) one or more polypeptides, one or more lipids, one or more small molecules, and one or more metal-containing compounds, ay) one or more polypeptides, one or more carbohydrates, one or more small molecules, and one or more metal-containing compounds, az) one or more nucleic acids, one or more lipids, one or more carbohydrates, and one or more small molecules, ba) one or more nucleic acids, one or more lipids, one or more carbohydrates, and one or more metal-containing compounds, bb) one or more nucleic acids, one or more lipids, one or more small molecules, and one or more metal-containing compounds, be) one or more nucleic acids, one or more carbohydrates, one or more small molecules, and one or more metal-containing compounds, bd) one or more lipids, one or more carbohydrates, one or more small molecules, and one or more metal-containing compounds, be) one or more polypeptides, one or more nucleic acids, one or more lipids, one or more carbohydrates, and one or more small molecules, bf) one or more polypeptides, one or more nucleic acids, one or more lipids, one or more carbohydrates, and one or more metal-containing compounds, bg) one or more polypeptides, one or more nucleic acids, one or more lipids, one or more small molecules, and one or more metal-containing compounds, bh) one or more polypeptides, one or more nucleic acids, one or more carbohydrates, one or more small molecules, and one or more metal-containing compounds, bi) one or more polypeptides, one or more lipids, one or more carbohydrates, one or more small molecules, and one or more metal-containing compounds, bj) one or more nucleic acids, one or more lipids, one or more carbohydrates, one or more small molecules, and one or more metal-containing compounds, or bk) one or more polypeptides, one or more nucleic acids, one or more lipids, one or more carbohydrates, one or more small molecules, and one or more metal-containing compounds.

In some embodiments, the complex is a polypeptide-nucleic acid complex. In some embodiments, the polypeptide-nucleic acid complex comprises a nucleic acid molecule that is complexed with a polypeptide via electrostatic attraction; a nucleic acid molecule wrapped around a polypeptide; DNA and a histone (nucleosome); a ribonucleoprotein (RNP); a ribosome; an enzyme telomerase; a vault ribonucleoprotein; ribonuclease P (RNase P); heterogeneous ribonucleoprotein particle (hnRNP); a small nuclear RNP (snRNP); or a chromosome comprising a protein.

The present subject matter is useful for delivering a great variety of polypeptide-complexes to cells, including a proteasome, a holoenzyme, an RNA polymerase, a DNA polymerase, a spliceosome, a vault cytoplasmic ribonucleoprotein, a small nuclear ribonucleic protein (snRNP), a telomerase, a nucleosome, a death signaling complex (DISC), a mammalian target of rapamycin complex 1 (mTORC1), a mammalian target of rapamycin complex 2 (mTORC2), or a class I phosphoinositide 3 kinase (Class I PI3K), histone-DNA complex, toll-like receptor (TLR)-agonist complex, transposase/transposon complex, tRNA ribosome complex, polypeptide-protease complex, or an enzyme-substrate complex.

In some embodiments, the ribonucleoprotein complex is a RNA-induced silencing complex (RISC). RISC is a catalytically active protein-RNA complex that is an important mediator of RNA interference (RNAi). RISC incorporates a strand of a double-stranded RNA (dsRNA) fragment, such as small interfering RNA (siRNA) or microRNA (miRNA). The strand acts as a template for RISC to recognize a complementary messenger RNA (mRNA) transcript. Argonaute, a protein component of RISC, subsequently activates and cleaves the mRNA.

In some embodiments, the ribonucleoprotein complex is a ribosome. Ribosomes consist of small and large ribosomal subunits, with each subunit composed of one or more ribosomal RNA (rRNA) molecules and a variety of proteins. Together, the ribosome complex mediates the translation of mRNA into polypeptide. In some embodiments, the invention provides methods of improved translation of an mRNA comprising passing a cell suspension through a constriction thereby allowing uptake of a complex comprising a mRNA and a ribosome, wherein the cell suspension is contacted with the mRNA-ribosome complex before, during or after passage of the cell suspension through the constriction. In some aspects, translation with the mRNA-ribosome complex is improved (e.g., translated more rapidly or translated to a greater extent) compared to delivery of mRNA alone to the cell.

In some embodiments, the complex is a transposase bound to target DNA. In some embodiments, transposase enzyme-target DNA complexes are delivered to mediate nucleic acid integration of the target DNA into the cell.

In some embodiments, the complex is a transcription factor complex. In some embodiments, the transcription factor complex consists of a transcription factor bound to a preinitiation complex, a large complex of proteins and RNA polymerase which is necessary for modulating gene transcription.

Exemplary proteins or polypeptides for use in the complex include, without limitation, a therapeutic protein, antibody, growth factor or inducer, fusion protein, antigen, synthetic protein, reporter marker, or selectable marker.

In some embodiments, polypeptide-nucleic acid complexes are not a complex of clustered regularly interspaced short palindromic repeats (CRISPR)-Cas9 (e.g., not a complex of a Cas9 protein and a guide RNA). In some embodiments, the complex of two or more molecules is not a complex used for gene editing selected from a zinc-finger nuclease (ZFN), transcription activator-like effector nuclease (TALEN), mega nuclease, or CRE recombinase.

In some embodiments, the polypeptide for use in the complex is a reporter or a selectable marker. Exemplary reporter markers include, without limitation, green fluorescent protein (GFP), red fluorescent protein (RFP), auquorin, beta-galactosidase, Uroporphyrinogen (urogen) III methyltransferase (UMT), and luciferase. Exemplary selectable markers include, without limitation, Blasticidin, G418/Geneticin, Hygromycin B, Puromycin, Zeocin, Adenine Phosphoribosyltransferase, and thymidine kinase.

In some embodiments, the polypeptide for use in the complex is a member of a high affinity binding pair, such as streptavidin or a variant thereof with high affinity for biotin. High affinity binding pairs comprising a polypeptide member are well known in the art, and any such polypeptide is contemplated for use in the compositions and methods described herein.

In some embodiments, the polypeptide for use in the complex is an antigen, e.g., a disease-associated antigen such as a tumor antigen, viral antigen, bacterial antigen, or fungal antigen. In some embodiments, the antigen comprises a whole, full-length (or un-processed) protein antigen, e.g., a protein or peptide that exceeds a length of 7, 8, 9, or 10 amino acids. In some embodiments, the antigen is a protein fragment, such as an MHC-restricted protein fragment, e.g., a peptide capable of associating with an MHC molecule to form a peptide/MHC complex. Exemplary antigens include, for example, the HPV16 E7 synthetic long peptide (SLP).

In some embodiments, the complex comprises an antigen associated with a carrier protein. Examples of suitable carrier proteins include proteins normally found in blood or plasma, which include, but are not limited to, albumin, immunoglobulin, including IgA, lipoproteins, apolipoprotein B, α-acid glycoprotein, β-2-macroglobulin, thyroglobulin, transferrin, fibronectin, factor VII, factor VIII, factor IX, factor X, and the like. In some embodiments, the carrier protein is a non-blood protein, such as casein, α-lactalbumin, or β-lactoglobulin. The carrier proteins may either be natural in origin or synthetically prepared. In some embodiments, the carrier comprises albumin, such as human serum albumin (HSA) or mouse serum albumin (MSA). Other albumins are contemplated, such as bovine serum albumin. Use of such non-human albumins could be appropriate, for example, in the context of use in non-human mammals, such as veterinary animals (including domestic pets and agricultural animals).

In some embodiments, the complex comprises an antigen associated with an adjuvant. Adjuvants are well known in the art, and any adjuvant that can associate with the antigen to form a complex is contemplated for use in the compositions and methods described herein.

In some embodiments, the complex comprises an antigen associated with a nanoparticle. Nanoparticles are well known in the art, and any nanoparticle that can associate with the antigen to form a complex is contemplated for use in the compositions and methods described herein (*see,* for example, Taki, A., & Smooker, P. (2015). Vaccines, 3(3), 638-661). Exemplary nanoparticles include, without limitation, inorganic nanoparticles, liposome nanoparticles, virus-like nanoparticles, and polymeric nanoparticles. Inorganic nanoparticles include, without limitation, nanoparticles comprising iron or silica. Liposome nanoparticles include, without limitation, conventional liposomes, sterically-stabilized liposomes, ligand-targeted liposomes, and combinations thereof (*see,* for example, Sercombe, L., et al. (2015). Frontiers in pharmacology, 6). For examples of virus-like nanoparticles see, for example, Plummer, E. M., & Manchester, M. (2011). Wiley Interdisciplinary Reviews: Nanomedicine and Nanobiotechnology, 3(2), 174-196. Polymeric nanoparticles include, without limitation, nanoparticles comprising chitosan, PLGA, or PEI (*see,* for example, Bolhassani, A., et al. (2014) Human Vaccines & Immunotherapeutics, 10:2, 321-332).

In some embodiments, the polypeptide for use in the complex is a toxin, including for example the bicyclic heptapeptide phalloidin.

In some embodiments, the polypeptide for use in the complex is an antibody. In some embodiments, the antibody is a full-length antibody or an antibody fragment. Antibodies for use in the present disclosure include, without limitation, antibody variants, labeled antibodies, antibody fragments such as Fab or F(ab)₂ fragments, single-domain antibodies, single-chain antibodies, multi-specific antibodies, antibody fusion proteins, and immunoadhesins. The antibodies may be any isotype known in the art, including IgA, IgG, IgE, IgD, or IgM.

Exemplary nucleic acids for use in the complex include, without limitation, recombinant nucleic acids, DNA, recombinant DNA, cDNA, genomic DNA, RNA, siRNA, mRNA, saRNA, miRNA, IncRNA, tRNA, and shRNA. In some embodiments, the nucleic acid is homologous to a nucleic acid in the cell. In some embodiments, the nucleic acid is heterologous to a nucleic acid in the cell. In some embodiments, the nucleic acid is a therapeutic nucleic acid. In some embodiments, the nucleic acid encodes a therapeutic polypeptide. In some embodiments, the nucleic acid encodes a reporter or a selectable marker. Exemplary reporter markers include, without limitation, green fluorescent protein (GFP), red fluorescent protein (RFP), auquorin, beta-galactosidase, Uroporphyrinogen (urogen) III methyltransferase (UMT), and luciferase. Exemplary selectable markers include, without limitation, Blasticidin, G418/Geneticin, Hygromycin B, Puromycin, Zeocin, Adenine Phosphoribosyltransferase, and thymidine kinase. In some embodiments, the nucleic acid encodes a growth factor or inducer.

Exemplary small molecules for use on the complex include, without limitation, biotin, fluorescent markers, dyes, pharmaceutical agents, metabolities, or radionucleotides. In some embodiments, the pharmaceutical agent is a therapeutic drug and/or cytotoxic agent.

Exemplary metal-containing compounds for use in the complex include silver, gold, platinum, copper, iron, iron oxide, and manganese. In some embodiments, the metal compound is a nanoparticle. In some embodiments, the nanoparticle is magnetic.

In some embodiments of the device and methods described herein, passage of stem cells or progenitor cells such as induced pluripotent stem cells (iPSCs) through a constriction channel does not induce differentiation, but does reliably induce uptake of complexes into the cell. For example, differentiation factor complexes are introduced into such cells. After uptake of differentiation factor complexes, the cells proceed on a differentiation pathway dictated by the introduced factor without complications associated with the method by which the factor(s) was introduced into the cell.

In some embodiments, the complex to deliver is purified. In some embodiments, the complex is at least about 20% by weight (dry weight) the complex of interest. In some embodiments, the purified complex is at least about 30%, 40%, 50%, 60%, 70%, 80%, 90%, or 99% the complex of interest. In some embodiments, the purified complex is at least about 90%, 91%, 92%, 93%, 94%, 95%, 98%, 99%, or 100% (w/w) the complex of interest. Purity is determined by any known methods, including, without limitation, column chromatography, thin layer chromatography, HPLC analysis, NMR, mass spectrometry, or SDS-PAGE. Purified DNA or RNA is defined as DNA or RNA that is free of exogenous nucleic acids, carbohydrates, and lipids.

### IV. CELL SUSPENSIONS

In some aspects, the invention provides methods to deliver complexes of two or more molecules into cells by passing a cell suspension through a constriction and contacting the cell suspension with the complex before, during or after passing the cell suspension through the constriction. In some embodiments, the cell suspension comprises animal cells. In some embodiments, the cell suspension comprises frog, chicken, insect, or nematode cells. In some embodiments, the cell suspension comprises mammalian cells. In some embodiments, the cell is a monkey, mouse, dog, cat, horse, rat, sheep, goat or rabbit cell. In some embodiments, the cell is a human cell.

In some embodiments, the cell suspension comprises a cell comprising a cell wall. In some embodiments, the cell is a plant, yeast, fungal, algal, or bacterial cell. In some embodiments, the cell is a plant cell. In some embodiments, the plant cell is a crop, model, ornamental, vegetable, leguminous, conifer, or grass plant cell. In some embodiments, the cell is a yeast cell. In some embodiments, the yeast cell is a *Candida* or *Saccharomyces* cell. In some embodiments, the cell is a fungal cell. In some embodiments, the fungal cell is an *Aspergillus* or *Penicillium* cell. In some embodiments, the cell is an algal cell. In some embodiments, the algal cell is a *Chlamydomonas, Dunaliella,* or *Chlorella* cell. In some embodiments, the cell suspension comprises bacterial cells. In some embodiments, the bacterial cell is a gram-positive bacterial cell. Gram-positive bacteria have a cell wall comprising a thick peptidoglycan layer. In some embodiments, the bacterial cell is a gram-negative bacterial cell. Gram-negative bacterial have a cell wall comprising a thin peptidoglycan layer between an inner cytoplasmic cell membrane and an outer membrane. In some embodiments, the bacterial cell is a *Streptococcus, Escherichia, Enterobacter, Bacillus, Pseudomonas, Klebsiella,* or *Salmonella* cell.

The cell suspension may be a mixed or purified population of cells. In some embodiments, the cell suspension is a mixed cell population, such as whole blood, lymph, and/or peripheral blood mononuclear cells (PBMCs). In some embodiments, the cell suspension is a purified cell population. In some embodiments, the cell is a primary cell or a cell line cell. In some embodiments, the cell is a blood cell. In some embodiments, the blood cell is an immune cell. In some embodiments, the immune cell is a lymphocyte. In some embodiments, the immune cell is a T cell, B cell, natural killer (NK) cell, dendritic cell (DC), NKT cell, mast cell, monocyte, macrophage, basophil, eosinophil, neutrophil, or DC2.4 dendritic cell. In some embodiments, the immune cell is a primary human T cell. In some embodiments, the blood cell is a red blood cell. In some embodiments, the cell is a cancer cell. In some embodiments, the cancer cell is a cancer cell line cell, such as a HeLa cell. In some embodiments, the cancer cell is a tumor cell. In some embodiments, the cancer cell is a circulating tumor cell (CTC). In some embodiments, the cell is a stem cell. Exemplary stem cells include, without limitation, induced pluripotent stem cells (iPSCs), embryonic stem cells (ESCs), liver stem cells, cardiac stem cells, neural stem cells, and hematopoietic stem cells. In some embodiments, the cell is a fibroblast cell, such as a primary fibroblast or newborn human foreskin fibroblast (Nuff cell). In some embodiments, the cell is an immortalized cell line cell, such as a HEK293 cell or a CHO cell. In some embodiments, the cell is a skin cell. In some embodiments, the cell is a reproductive cell such as an oocyte, ovum, or zygote. In some embodiments, the cell is a neuron. In some embodiments, the cell is a cluster of cells, such as an embryo, given that the cluster of cells is not disrupted when passing through the pore.

The composition of the cell suspension (e.g., osmolarity, salt concentration, serum content, cell concentration, pH, etc.) can impact delivery of the complex. In some embodiments, the suspension comprises whole blood. Alternatively, the cell suspension is a mixture of cells in a physiological saline solution or physiological medium other than blood. In some embodiments, the cell suspension comprises an aqueous solution. In some embodiments, the aqueous solution comprises cell culture medium, PBS, salts, sugars, growth factors, animal derived products, bulking materials, surfactants, lubricants, vitamins, polypeptides, and/or an agent that impacts actin polymerization. In some embodiments, the cell culture medium is DMEM, OptiMEM, IMDM, or RPMI. Additionally, solution buffer can include one or more lubricants (pluronics or other surfactants) that can be designed to reduce or eliminate clogging of the surface and improve cell viability. Exemplary surfactants include, without limitation, poloxamer, polysorbates, sugars such as mannitol, animal derived serum, and albumin protein.

In some configurations with certain types of cells, the cells can be incubated in one or more solutions that aid in the delivery of the complex to the interior of the cell. In some embodiments, the aqueous solution comprises an agent that impacts actin polymerization. In some embodiments, the agent that impacts actin polymerization is Latrunculin A, Cytochalasin, and/or Colchicine. For example, the cells can be incubated in a depolymerization solution such as Lantrunculin A (0.lµg/ml) for 1 hour prior to delivery to depolymerize the actin cytoskeleton. As an additional example, the cells can be incubated in 10µM Colchicine (Sigma) for 2 hours prior to delivery to depolymerize the microtubule network.

The viscosity of the cell suspension can also impact the methods disclosed herein. In some embodiments, the viscosity of the cell suspension ranges from about 8.9 × 10⁻⁴ Pa·s to about 4.0 × 10⁻³ Pa·s or any value or range of values therebetween. In some embodiments, the viscosity ranges between any one of about 8.9 × 10⁻⁴ Pa·s to about 4.0 × 10⁻³ Pa s, about 8.9 × 10⁻⁴ Pa·s to about 3.0 × 10⁻³ Pa s, about 8.9 × 10⁻⁴ Pa·s to about 2.0 × 10⁻³ Pa s, or about 8.9 × 10⁻³ Pa·s to about 1.0 × 10⁻³ Pa s. In some embodiments, the viscosity ranges between any one of about 0.89 cP to about 4.0 cP, about 0.89 cP to about 3.0 cP, about 0.89 cP to about 2.0 cP, or about 0.89 cP to about 1.0 cP. In some embodiments, a shear thinning effect is observed, in which the viscosity of the cell suspension decreases under conditions of shear strain. Viscosity can be measured by any method known in the art, including without limitation, viscometers, such as a glass capillary viscometer, or rheometers. A viscometer measures viscosity under one flow condition, while a rheometer is used to measure viscosities which vary with flow conditions. In some embodiments, the viscosity is measured for a shear thinning solution such as blood. In some embodiments, the viscosity is measured between about 0°C and about 45°C. For example, the viscosity is measured at room temperature (e.g., about 20°C), physiological temperature (e.g., about 37°C), higher than physiological temperature (e.g., greater than about 37°C to 45°C or more), reduced temperature (e.g., about 0°C to about 4°C), or temperatures between these exemplary temperatures.

### V. MICROFLUIDIC CHANNELS TO PROVIDE CELL-DEFORMING CONSTRICTIONS

In some aspects, the invention provides methods to deliver complexes of two or more molecules into cells by passing a cell suspension through a constriction and contacting the cell suspension with the complex before, during or after passing the cell suspension through the constriction. In some embodiments, the constriction is contained within a microfluidic channel. In some embodiments, multiple constrictions can be placed in parallel and/or in series within the microfluidic channel. Exemplary microfluidic channels containing cell-deforming constrictions for use in the methods disclosed herein are described in WO2013059343.

In some embodiments, the microfluidic channel includes a lumen and is configured such that a cell suspended in a buffer can pass through, wherein the microfluidic channel includes a constriction. The microfluidic channel can be made of any one of a number of materials, including silicon, metal (e.g., stainless steel), plastic (e.g., polystyrene), ceramics, glass, crystalline substrates, amorphous substrates, or polymers (e.g., Poly-methyl methacrylate (PMMA), PDMS, Cyclic Olefin Copolymer (COC), etc.). Fabrication of the microfluidic channel can be performed by any method known in the art, including dry etching, wet etching, photolithography, injection molding, laser ablation, or SU-8 masks.

In some embodiments, the constriction within the microfluidic channel includes an entrance portion, a centerpoint, and an exit portion. In some embodiments, the length, depth, and width of the constriction within the microfluidic channel can vary. In some embodiments, the diameter of the constriction within the microfluidic channel is a function of the diameter of the cell comprising a cell wall. In some embodiments, the diameter of the constriction within the microfluidic channel is about 20% to about 99% of the diameter of the cell. In some embodiments, the constriction size is about 20%, about 30%, about 40%, about 50%, about 60%, about 70%, about 80%, about 90%, or about 99% of the cell diameter. The cross-section of the channel, the entrance portion, the centerpoint, and the exit portion can also vary. For example, the cross-sections can be circular, elliptical, an elongated slit, square, hexagonal, or triangular in shape. The entrance portion defines a constriction angle, wherein the constriction angle is optimized to reduce clogging of the channel. The angle of the exit portion can vary as well. For example, the angle of the exit portion is configured to reduce the likelihood of turbulence that can result in non-laminar flow. In some embodiments, the walls of the entrance portion and/or the exit portion are linear. In other embodiments, the walls of the entrance portion and/or the exit portion are curved.

### VI. SURFACE HAVING PORES TO PROVIDE CELL-DEFORMING CONSTRICTIONS

In some aspects, the invention provides methods to deliver complexes of two or more molecules into cells by passing a cell suspension through a constriction and contacting the cell suspension with the complex before, during or after passing the cell suspension through the constriction. In some embodiments, the pore is contained in a surface. Exemplary surfaces having pores for use in the methods disclosed herein are described in U.S. Provisional Application 62/214,820, filed 09/04/2015.

The surfaces as disclosed herein can be made of any one of a number of materials and take any one of a number of forms. In some embodiments, the surface is a filter. In some embodiments, the surface is a membrane. In some embodiments, the filter is a tangential flow filter. In some embodiments, the surface is a sponge or sponge-like matrix. In some embodiments, the surface is a matrix.

In some embodiments, the surface is a tortuous path surface. In some embodiments, the tortuous path surface comprises cellulose acetate. In some embodiments, the surface comprises a material selected from, without limitation, synthetic or natural polymers, polycarbonate, silicon, glass, metal, alloy, cellulose nitrate, silver, cellulose acetate, nylon, polyester, polyethersulfone, Polyacrylonitrile (PAN), polypropylene, PVDF, polytetrafluorethylene, mixed cellulose ester, porcelain, and ceramic.

The surface disclosed herein can have any shape known in the art; e.g. a 3-dimensional shape. The 2-dimensional shape of the surface can be, without limitation, circular, elliptical, round, square, star-shaped, triangular, polygonal, pentagonal, hexagonal, heptagonal, or octagonal. In some embodiments, the surface is round in shape. In some embodiments, the surface 3-dimensional shape is cylindrical, conical, or cuboidal.

The surface can have various cross-sectional widths and thicknesses. In some embodiments, the surface cross-sectional width is between about 1mm and about 1m or any cross-sectional width or range of cross-sectional widths therebetween. In some embodiments, the surface has a defined thickness. In some embodiments, the surface thickness is uniform. In some embodiments, the surface thickness is variable. For example, in some embodiments, portions of the surface are thicker or thinner than other portions of the surface. In some embodiments, the surface thickness varies by about 1% to about 90% or any percentage or range of percentages therebetween. In some embodiments, the surface is between about 0.01µm to about 5mm thick or any thickness or range of thicknesses therebetween.

In some embodiments, the constriction is a pore or contained within a pore. The cross-sectional width of the pores is related to the type of cell to be treated. In some embodiments, the pore size is a function of the diameter of the cell of cluster of cells to be treated. In some embodiments, the pore size is such that a cell is perturbed upon passing through the pore. In some embodiments, the pore size is less than the diameter of the cell. In some embodiments, the pore size is about 20% to about 99% of the diameter of the cell. In some embodiments, the pore size is about 20%, about 30%, about 40%, about 50%, about 60%, about 70%, about 80%, about 90%, or about 99% of the cell diameter. Optimal pore size can vary based upon the application and/or cell type. In some embodiments, the pore size is about 0.4 µm, about 1 µm, about 2 µm, about 3 µm, about 4 µm, about 5 µm, about 6 µm, about 7 µm, about 8 µm, about 9 µm, about 10 µm, about 11 µm, about 12 µm, about 13 µm, or about 14 µm.

The entrances and exits of the pore passage may have a variety of angles. The pore angle can be selected to minimize clogging of the pore while cells are passing through. In some embodiments, the flow rate through the surface is between about 0.001 mL/cm²/sec to about 100 L/cm²/sec or any rate or range of rates therebetween. For example, the angle of the entrance or exit portion can be between about 0 and about 90 degrees. In some embodiments, the pores have identical entrance and exit angles. In some embodiments, the pores have different entrance and exit angles. In some embodiments, the pore edge is smooth, e.g. rounded or curved. A smooth pore edge has a continuous, flat, and even surface without bumps, ridges, or uneven parts. In some embodiments, the pore edge is sharp. A sharp pore edge has a thin edge that is pointed or at an acute angle. In some embodiments, the pore passage is straight. A straight pore passage does not contain curves, bends, angles, or other irregularities. In some embodiments, the pore passage is curved. A curved pore passage is bent or deviates from a straight line. In some embodiments, the pore passage has multiple curves, e.g. about 2, 3, 4, 5, 6, 7, 8, 9, 10 or more curves.

The pores can have any shape known in the art, including a 2-dimensional or 3-dimensional shape. The pore shape (e.g., the cross-sectional shape) can be, without limitation, circular, elliptical, round, square, star-shaped, triangular, polygonal, pentagonal, hexagonal, heptagonal, and octagonal. In some embodiments, the cross-section of the pore is round in shape. In some embodiments, the 3-dimensional shape of the pore is cylindrical or conical. In some embodiments, the pore has a fluted entrance and exit shape. In some embodiments, the pore shape is homogenous (i.e. consistent or regular) among pores within a given surface. In some embodiments, the pore shape is heterogeneous (i.e. mixed or varied) among pores within a given surface.

The surfaces described herein can have a range of total pore numbers. In some embodiments, the pores encompass about 10% to about 80% of the total surface area. In some embodiments, the surface contains about 1.0 × 10⁵ to about 1.0 × 10³⁰ total pores or any number or range of numbers therebetween. In some embodiments, the surface comprises between about 10 and about 1.0 × 10¹⁵ pores per mm² surface area.

The pores can be distributed in numerous ways within a given surface. In some embodiments, the pores are distributed in parallel within a given surface. In one such example, the pores are distributed side-by-side in the same direction and are the same distance apart within a given surface. In some embodiments, the pore distribution is ordered or homogeneous. In one such example, the pores are distributed in a regular, systematic pattern or are the same distance apart within a given surface. In some embodiments, the pore distribution is random or heterogeneous. In one such example, the pores are distributed in an irregular, disordered pattern or are different distances apart within a given surface. In some embodiments, multiple surfaces are distributed in series. The multiple surfaces can be homogeneous or heterogeneous in surface size, shape, and/or roughness. The multiple surfaces can further contain pores with homogeneous or heterogeneous pore size, shape, and/or number, thereby enabling the simultaneous delivery of a range of complexes into different cell types.

In some embodiments, an individual pore has a uniform width dimension (i.e. constant width along the length of the pore passage). In some embodiments, an individual pore has a variable width (i.e. increasing or decreasing width along the length of the pore passage). In some embodiments, pores within a given surface have the same individual pore depths. In some embodiments, pores within a given surface have different individual pore depths. In some embodiments, the pores are immediately adjacent to each other. In some embodiments, the pores are separated from each other by a distance. In some embodiments, the pores are separated from each other by a distance of about 0.001 µm to about 30 mm or any distance or range of distances therebetween.

In some embodiments, the surface is coated with a material. The material can be selected from any material known in the art, including, without limitation, Teflon, an adhesive coating, surfactants, proteins, adhesion molecules, antibodies, anticoagulants, factors that modulate cellular function, nucleic acids, lipids, carbohydrates, or transmembrane proteins. In some embodiments, the surface is coated with polyvinylpyrrolidone. In some embodiments, the material is covalently attached to the surface. In some embodiments, the material is non-covalently attached to the surface. In some embodiments, the surface molecules are released at the cells pass through the pores.

In some embodiments, the surface has modified chemical properties. In some embodiments, the surface is hydrophilic. In some embodiments, the surface is hydrophobic. In some embodiments, the surface is charged. In some embodiments, the surface is positively and/or negatively charged. In some embodiments, the surface can be positively charged in some regions and negatively charged in other regions. In some embodiments, the surface has an overall positive or overall negative charge. In some embodiments, the surface can be any one of smooth, electropolished, rough, or plasma treated. In some embodiments, the surface comprises a zwitterion or dipolar compound. In some embodiments, the surface is plasma treated.

In some embodiments, the surface is contained within a larger module. In some embodiments, the surface is contained within a syringe, such as a plastic or glass syringe. In some embodiments, the surface is contained within a plastic filter holder. In some embodiments, the surface is contained within a pipette tip.

### VII. CELL PERTURBATIONS

In some embodiments, the invention provides methods for delivery of a complex to a cell by passing a cell suspension through a constriction, wherein the constriction deforms the cell thereby causing a perturbation of the cell such that a complex enters the cell, wherein the perturbation in the cell is a breach in the cell that allows material from outside the cell to move into the cell (e.g., a hole, tear, cavity, aperture, pore, break, gap, perforation). The deformation can be caused by, for example, pressure induced by mechanical strain and/or shear forces. In some embodiments, the perturbation is a perturbation within the cell wall. In some embodiments, the perturbation is a perturbation within the cell membrane. In some embodiments, the perturbation is transient. In some embodiments, the cell perturbation lasts from about 1.0×10⁻⁹ seconds to about 2 hours, or any time or range of times therebetween. In some embodiments, the cell perturbation lasts for about 1.0×10⁻⁹second to about 1 second, about 1 second to about 1 minute, or about 1 minute to about 1 hour. In some embodiments, the cell perturbation lasts for between any one of about 1.0×10⁻⁹ to about 1.0×10⁻¹, about 1.0×10⁻⁹ to about 1.0×10⁻², about 1.0×10⁻⁹ to about 1.0×10⁻³, about 1.0×10⁻⁹ to about 1.0×10⁻⁴, about 1.0×10⁻⁹ to about 1.0×10⁻⁵, about 1.0×10⁻⁹ to about 1.0×10⁻⁶, about 1.0×10⁻⁹ to about 1.0×10⁻⁷, or about 1.0×10⁻⁹ to about 1.0×10⁻⁸ seconds. In some embodiment, the cell perturbation lasts for any one of about 1.0×10⁻⁸ to about 1.0×10⁻¹, about 1.0×10⁻⁷ to about 1.0×10⁻¹, about 1.0×10⁻⁶ to about 1.0×10⁻¹, about 1.0×10⁻⁵ to about 1.0×10⁻¹, about 10×10⁻⁴ to about 1.0×10⁻¹, about 1.0×10⁻³ to about 10×10⁻¹, or about 10×10⁻² to about 1.0×10⁻¹ seconds. The cell perturbations (e.g., pores or holes) created by the methods described herein are not formed as a result of assembly of polypeptide subunits to form a multimeric pore structure such as that created by complement or bacterial hemolysins.

As the cell passes through the constriction, the constriction temporarily imparts injury to the cell membrane that causes passive diffusion of material through the perturbation. In some embodiments, the cell is only deformed for a brief period of time, on the order of 100 µs to minimize the chance of activating apoptotic pathways through cell signaling mechanisms, although other durations are possible (e.g., ranging from nanoseconds to hours). In some embodiments, the cell is deformed for about 1.0 ×10⁻⁹ seconds to about 2 hours, or any time or range of times therebetween. In some embodiments, the cell is deformed for about 1.0×10⁻⁹ second to about 1 second, about 1 second to about 1 minute, or about 1 minute to about 1 hour. In some embodiments, the cell is deformed for between any one of about 1.0×10⁻⁹ to about 1.0×10⁻¹, about 1.0×10⁻⁹ to about 1.0×10⁻², about 1.0×10⁻⁹ to about 1.0×10⁻³, about 1.0×10⁻⁹ to about 1.0×10⁻⁴, about 1.0×10⁻⁹ to about 1.0×10⁻⁵, about 1.0×10⁻⁹ to about 1.0×10⁻⁶, about 1.0×10⁻⁹ to about 1.0×10⁻⁷, or about 1.0×10⁻⁹ to about 1.0×10⁻⁸ seconds. In some embodiment, the cell is deformed for any one of about 1.0×10⁻⁸ to about 1.0×10⁻¹, about 1.0×10⁻⁷ to about 1.0×10⁻¹, about 1.0×10⁻⁶ to about 1.0×10⁻¹, about 1.0×10⁻⁵ to about 1.0×10⁻¹, about 1.0×10⁻⁴ to about 1.0×10⁻¹, about 1.0×10⁻³ to about 1.0×10⁻¹, or about 10×10⁻² to about 1.0×10⁻¹ seconds. In some embodiments, deforming the cell includes deforming the cell for a time ranging from, without limitation, about 1 µs to at least about 750 µs, e.g., at least about 1µs, 10 µs, 50µs, 100 µs, 500 µs, or 750 µs.

In some embodiments, the passage of the complex into the cell occurs simultaneously with the cell passing through the constriction and/or the perturbation of the cell. In some embodiments, passage of the complex into the cell occurs after the cell passes through the constriction. In some embodiments, passage of the complex into the cell occurs on the order of minutes after the cell passes through the constriction. In some embodiments, the passage of the complex into the cell occurs from about 1.0×10⁻² seconds to at least about 30 minutes after the cell passes through the constriction. For example, the passage of the complex into the cell occurs from about 1.0×10⁻² seconds to about 1 second, about 1 second to about 1 minute, or about 1 minute to about 30 minutes after the cell passes through the constriction. In some embodiments, the passage of the complex into the cell occurs about 1.0×10⁻² seconds to about 10 minutes, about 10×10⁻² seconds to about 5 minutes, about 1.0×10⁻² seconds to about 1 minute, about 1.0×10⁻² seconds to about 50 seconds, about 1.0×10⁻² seconds to about 10 seconds, about 1.0×10⁻² seconds to about 1 second, or about 1.0×10⁻² seconds to about 0.1 second after the cell passes through the constriction. In some embodiments, the passage of the complex into the cell occurs about 1.0×10⁻¹ seconds to about 10 minutes, about 1 second to about 10 minutes, about 10 seconds to about 10 minute, about 50 seconds to about 10 minutes, about 1 minute to about 10 minutes, or about 5 minutes to about 10 minutes after the cell passes through the constriction. In some embodiments, a perturbation in the cell after it passes through the constriction is corrected within the order of about five minutes after the cell passes through the constriction.

In some embodiments, the cell viability after passing through a constriction is about 5% to about 100%. In some embodiments, the cell viability after passing through the constriction is at least about 5%, 10%, 20%, 30%, 40%, 50%, 60%, 70%, 75%, 80%, 85%, 90%, 95%, or 99%. In some embodiments, the cell viability is measured from about 1.0×10⁻² seconds to at least about 10 days after the cell passes through the constriction. For example, the cell viability is measured from about 1.0×10⁻² seconds to about 1 second, about 1 second to about 1 minute, about 1 minute to about 30 minutes, or about 30 minutes to about 2 hours after the cell passes through the constriction. In some embodiments, the cell viability is measured about 1.0×10⁻² seconds to about 2 hours, about 1.0×10⁻² seconds to about 1 hour, about 1.0×10⁻² seconds to about 30 minutes, about 1.0×10⁻² seconds to about 1 minute, about 1.0×10⁻² seconds to about 30 seconds, about 1.0×10⁻² seconds to about 1 second, or about 1.0×10⁻² seconds to about 0.1 second after the cell passes through the constriction. In some embodiments, the cell viability is measured about 1.5 hours to about 2 hours, about 1 hour to about 2 hours, about 30 minutes to about 2 hours, about 15 minutes to about 2 hours, about 1 minute to about 2 hours, about 30 seconds to about 2 hours, or about 1 second to about 2 hours after the cell passes through the constriction. In some embodiments, the cell viability is measured about 2 hours to about 5 hours, about 5 hours to about 12 hours, about 12 hours to about 24 hours, or about 24 hours to about 10 days after the cell passes through the constriction.

### VIII. DELIVERY PARAMETERS

A number of parameters may influence the delivery of a complex to a cell by the methods described herein. In some embodiments, the cell suspension is contacted with the complex before, concurrently, or after passing through the constriction. The cell may pass through the constriction suspended in a solution that includes the complex to deliver, although the complex can be added to the cell suspension after the cells pass through the constriction. In some embodiments, the complex to be delivered is coated on the constriction.

Examples of parameters that may influence the delivery of the complex into the cell include, but are not limited to, the dimensions of the constriction, the entrance angle of the constriction, the surface properties of the constrictions (e.g., roughness, chemical modification, hydrophilic, hydrophobic, etc.), the operating flow speeds (e.g., cell transit time through the constriction), the cell concentration, the concentration of the complex in the cell suspension, and the amount of time that the cell recovers or incubates after passing through the constrictions can affect the passage of the delivered complex into the cell. Additional parameters influencing the delivery of the complex into the cell can include the velocity of the cell in the constriction, the shear rate in the constriction, the viscosity of the cell suspension, the velocity component that is perpendicular to flow velocity, and time in the constriction. Such parameters can be designed to control delivery of the complex. In some embodiments, the cell concentration ranges from about 10 to at least about 10¹² cells/ml or any concentration or range of concentrations therebetween. In some embodiments, delivery complex concentrations can range from about 10 ng/ml to about 1g/mL or any concentration or range of concentrations therebetween. In some embodiments, delivery complex concentrations can range from about 1pM to at least about 2M or any concentration or range of concentrations therebetween.

The temperature used in the methods of the present disclosure can be adjusted to affect complex delivery and cell viability as well as complex stability. In some embodiments, the method is performed between about -5°C and about 45°C. For example, the methods can be carried out at room temperature (e.g., about 20°C), physiological temperature (e.g., about 37°C), higher than physiological temperature (e.g., greater than about 37°C to 45°C or more), or reduced temperature (e.g., about -5°C to about 4°C), or temperatures between these exemplary temperatures. In some embodiments, the cell suspension is contacted with the complex of two or more molecules at a temperature that does not result in substantial dissociation of the complex of molecules to be delivered to the cell. In some embodiments, the cell suspension is contacted with the complex of two or more molecules at a temperature that results in less than about 10%, 20%, 30%, 40%, 50%, 60%, 70%, 80%, or 90% dissociation of the complex of molecules to be delivered to the cell.

Various methods can be utilized to drive the cells through the constrictions. For example, pressure can be applied by a pump on the entrance side (e.g., gas cylinder, or compressor), a vacuum can be applied by a vacuum pump on the exit side, capillary action can be applied through a tube, and/or the system can be gravity fed. Displacement based flow systems can also be used (e.g., syringe pump, peristaltic pump, manual syringe or pipette, pistons, etc.). In some embodiments, the cells are passed through the constrictions by positive pressure or negative pressure. In some embodiments, the cells are passed through the constrictions by constant pressure or variable pressure. In some embodiments, pressure is applied using a syringe. In some embodiments, pressure is applied using a pump. In some embodiments, the pump is a peristaltic pump or a diaphragm pump. In some embodiments, pressure is applied using a vacuum. In some embodiments, the cells are passed through the constrictions by g-force. In some embodiments, the cells are passed through the constrictions by capillary pressure.

In some embodiments, fluid flow directs the cells through the constrictions. In some embodiments, the fluid flow is turbulent flow prior to the cells passing through the constriction. Turbulent flow is a fluid flow in which the velocity at a given point varies erratically in magnitude and direction. In some embodiments, the fluid flow through the constriction is laminar flow. Laminar flow involves uninterrupted flow in a fluid near a solid boundary in which the direction of flow at every point remains constant. In some embodiments, the fluid flow is turbulent flow after the cells pass through the constriction. The velocity at which the cells pass through the constrictions can be varied. In some embodiments, the cells pass through the constrictions at a uniform cell speed. In some embodiments, the cells pass through the constrictions at a fluctuating cell speed.

In other embodiments, a combination treatment is used to deliver the complexes, e.g., the methods described herein followed by exposure to an electric field downstream of the constriction. In some embodiments, the cell is passed through an electric field generated by at least one electrode after passing through the constriction. In some embodiments, the electric field assists in delivery of complexes to a second location inside the cell such as the cell nucleus. In some embodiments, one or more electrodes are in proximity to the cell-deforming constriction to generate an electric field. In some embodiments, the electric field is between about 0.1kV/m to about 100MV/m, or any number or range of numbers therebetween. In some embodiments, an integrated circuit is used to provide an electrical signal to drive the electrodes. In some embodiments, the cells are exposed to the electric field for a pulse width of between about 1ns to about 1s and a period of between about 100ns to about 10s or any time or range of times therebetween.

### IX. SYSTEMS AND KITS

In some aspects, the invention provides a system for delivery of a complex of two or more molecules into a cell, the system comprising a microfluidic channel comprising a constriction, a cell suspension comprising the cell, and the complex of two or more molecules; wherein the constriction is configured such that the cell can pass through the constriction wherein the constriction deforms the cell thereby causing a perturbation of the cell such that the complex of two or more molecules enters the cell. In other aspects, the invention provides a system for delivering a complex of two or more molecules into a cell, the system comprising a surface with pores, a cell suspension comprising the cell, and the complex of two or more molecules; wherein the surface with pores is configured such that the cell can pass through the pore wherein the pore deforms the cell thereby causing a perturbation of the cell such that the complex of two or more molecules enters the cell. In some embodiments, the surface is a filter or a membrane. In some embodiments of the above aspects, the system further comprises at least one electrode to generate an electric field. In some embodiments, the formation of the complex of molecules is reversible. In some embodiments, at least two or more molecules of the complex associate by noncovalent interactions. In some embodiments, the system is used to deliver into a cell any complex of two or more molecules as described herein. In some embodiments, the system is used to deliver a complex comprising two or more molecules into a cell by any of the methods described herein. The system can include any embodiment described for the methods disclosed above, including microfluidic channels or a surface having pores to provide cell-deforming constrictions, cell suspensions, cell perturbations, delivery parameters, compounds to alter or induce antibody production, and/or applications etc. In some embodiment, the cell-deforming constrictions are sized for delivery to antibody-producing cells to alter endogenous antibody production. In some embodiments, the cell-deforming constrictions are sized for delivery of a compound that induces de novo antibody production in a cell. In some embodiments, the delivery parameters, such as operating flow speeds, cell and compound concentration, velocity of the cell in the constriction, and the composition of the cell suspension (e.g., osmolarity, salt concentration, serum content, cell concentration, pH, etc.) are optimized for delivery of a complex of two or more molecules into the cell.

In some embodiments, the invention provides a cell comprising a complex of two or more molecules as described herein, wherein the complex of two or more molecules was delivered into the cell by any of the methods described herein.

Also provided are kits or articles of manufacture for use in delivering into a cell a complex of two or more molecules as described herein. In some embodiments, the kits comprise the compositions described herein (e.g. a microfluidic channel or surface containing pores, cell suspensions, and/or complexes of two or more molecules) in suitable packaging. Suitable packaging materials are known in the art, and include, for example, vials (such as sealed vials), vessels, ampules, bottles, jars, flexible packaging (e.g., sealed Mylar or plastic bags), and the like. These articles of manufacture may further be sterilized and/or sealed.

The present disclosure also provides kits comprising components of the methods described herein and may further comprise instruction(s) for performing said methods to deliver a complex of two or more molecules into a cell. The kits described herein may further include other materials, including other buffers, diluents, filters, needles, syringes, and package inserts with instructions for performing any methods described herein; e.g., instructions for delivering a complex of two or more molecules into a cell.

### X. EXEMPLARY EMBODIMENTS

1. A method for delivering a complex of two or more molecules into a cell, the method comprising passing a cell suspension through a constriction, wherein said constriction deforms the cell, thereby causing a perturbation of the cell such that the complex of molecules enters the cell, wherein said cell suspension is contacted with the complex of molecules.
2. The method of embodiment 1, wherein formation of the complex of molecules is reversible.
3. The method of embodiment 1 or 2, wherein at least two or more molecules of the complex associate by noncovalent interactions.
4. The method of any one of embodiments 1-3, wherein at least two molecules in the complex have a binding affinity in the complex ranging from about 1 µM to about 1 pM.
5. The method of any one of embodiments 1-4, wherein at least two molecules in the complex have a binding affinity in the complex ranging from about 1 µM to about 1 nM or from about 1 nM to about 1 pM.
6. The method of any one of embodiments 1-5, wherein the complex has a half-life in the cell suspension of about 1 minute to about 48 hours.
7. The method of embodiment 6, wherein the complex has a half-life in the cell suspension of about 1 minute to about 20 minutes, about 20 minutes to about 40 minutes, about 40 minutes to about 1 hour, about 1 hour to about 2 hours, about 2 hours to about 6 hours, about 6 hours to about 12 hours, about 12 hours to about 24 hours, about 24 hours to about 36 hours, or about 36 hours to about 48 hours.
8. The method of any one of embodiments 1-7, wherein the complex dissociates in the presence of a detergent.
9. The method of embodiment 8, wherein the complex dissociates in the presence of a detergent at a concentration of about 0.1% (w/v) to about 10% (w/v).
10. The method of embodiment 8 or 9, wherein the complex dissociates in the presence of a detergent at a concentration of about 0.1% (w/v) to about 1% (w/v), about 1% (w/v) to about 5% (w/v), or about 5% (w/v) to about 10% (w/v).
11. The method of any one of embodiments 1-10, wherein the cell suspension is contacted with the complex of molecules at a temperature ranging from about 0 °C to about 40 °C.
12. The method of embodiment 11, wherein the complex of molecules dissociates at a temperature greater than the temperature at which the cell suspension is contacted with the complex of molecules.
13. The method of embodiment 11 or 12, wherein the complex of molecules dissociates at a temperature of about 50 °C to about 70 °C.
14. The method of any one of embodiments 11-13, wherein the complex of molecules dissociates at a temperature of about 50 °C to about 60 °C, or about 60 °C to about 70 °C.
15. The method of any one of embodiments 1-14, wherein the cell suspension is contacted with the complex of molecules at an ionic strength ranging from about 50 mM to about 300 mM.
16. The method of embodiment 15, wherein the complex of molecules dissociates at an ionic strength greater than the ionic strength at which the cell suspension is contacted with the complex of molecules.
17. The method of embodiment 15 or 16, wherein the complex of molecules dissociates at an ionic strength of about 350 mM to about 1000 mM.
18. The method of embodiment 17, wherein the complex of molecules dissociates at an ionic strength of about 350 mM to about 400 mM, about 400 mM to about 500 mM, about 500 mM to about 600 mM, about 700 mM to about 800 mM, about 800 mM to about 900 mM, or about 900 mM to about 1000 mM.
19. The method of embodiment 15, wherein the complex of molecules dissociates at an ionic strength less than the ionic strength at which the cell suspension is contacted with the complex of molecules.
20. The method of embodiment 15 or 19, wherein the complex of molecules dissociates at an ionic strength of about 0 mM to about 50 mM.
21. The method of embodiment 20, wherein the complex of molecules dissociates at an ionic strength of about 0 mM to about 10 mM, about 10 mM to about 20 mM, about 20 mM to about 30 mM, about 30 mM to about 40 mM, or about 40 mM to about 50 mM.
22. The method of any one of embodiments 1-14, wherein the cell suspension is contacted with the complex of molecules at an osmolarity ranging from about 100 mOsm/L to about 500 mOsm/L.
23. The method of embodiment 22, wherein the complex of molecules dissociates at an osmolarity greater than the ionic strength at which the cell suspension is contacted with the complex of molecules.
24. The method of embodiment 22 or 23, wherein the complex of molecules dissociates at an osmolarity of about 600 mOsm/L to about 1000 mOsm/L.
25. The method of embodiment 24, wherein the complex of molecules dissociates at an osmolarity of about 600 mOsm/L to about 700 mOsm/L, about 700 mOsm/L to about 800 mOsm/L, about 800 mOsm/L to about 900 mOsm/L, or about 900 mOsm/L to about 1000 mOsm/L.
26. The method of embodiment 22, wherein the complex of molecules dissociates at an osmolarity less than the osmolarity at which the cell suspension is contacted with the complex of molecules.
27. The method of embodiment 22 or 26, wherein the complex of molecules dissociates at an osmolarity of about 0 mOsm/L to about 100 mOsm/L.
28. The method of embodiment 27, wherein the complex of molecules dissociates at an osmolarity of about 0 mOsm/L to about 20 mOsm/L, about 20 mOsm/L to about 20 mOsm/L, about 20 mOsm/L to about 40 mOsm/L, about 40 mOsm/L to about 60 mOsm/L, about 60 mOsm/L to about 80 mOsm/L, or about 80 mOsm/L to about 100 mOsm/L.
29. The method of any one of embodiments 1-28, wherein the cell suspension is contacted with the complex of molecules at a pH ranging from about 5.5 to about 8.5.
30. The method of embodiment 29, wherein the complex of molecules dissociates at a pH greater or lower than the pH at which the cell suspension is contacted with the complex of molecules.
31. The method of embodiment 29 or 30, wherein the complex of molecules dissociates at a pH of about 4.0 to about 5.5 or at a pH of about 8.5 to about 10.
32. The method of embodiment 31, wherein the complex of molecules dissociates at a pH of about 4.0 to about 4.5, about 4.5 to about 5.0, about 5.0 to about 5.5, about 8.5 to about 9.0, about 9.0 to about 9.5, or about 9.5 to about 10.0.
33. The method of any one of embodiments 1-32, wherein the shear force as the cell passes through the constriction ranges from about 1 kPa to about 10 kPa.
34. The method of embodiment 33, wherein the complex dissociates at a shear force of about 10 kPa to about 100 kPa.
35. The method of embodiment 33 or 34, wherein the complex dissociates at a shear force of about 10 kPa to about 25 kPa, about 25 kPa to about 50 kPa, about 50 kPa to about 75 kPa, or about 75 kPa to about 100 kPa.
36. The method of any one of embodiments 1-35, wherein the complex of molecules comprises a) one or more polypeptides, b) one or more nucleic acids, c) one or more lipids, d) one or more carbohydrates, e) one or more small molecules, f) one or more metal-containing compounds, g) one or more polypeptides and one or more nucleic acids, h) one or more polypeptides and one or more lipids, i) one or more polypeptides and one or more carbohydrates, j) one or more polypeptides and one or more small molecules, k) one or more polypeptides and one or more metal-containing compounds, 1) one or more nucleic acids and one or more lipids, m) one or more nucleic acids and one or more carbohydrates, n) one or more nucleic acids and one or more small molecules, o) one or more nucleic acids and one or more metal-containing compounds, p) one or more lipids and one or more carbohydrates, q) one or more lipids and one or more small molecules, r) one or more lipids and one or more metal-containing compounds, s) one or more carbohydrates and one or more small molecules, t) one or more carbohydrates and one or more metal-containing compounds, u) one or more small molecules and one or more metal-containing compounds, v) one or more polypeptides, one or more nucleic acids and one or more lipids, w) one or more polypeptides, one or more nucleic acids and one or more carbohydrate, x) one or more polypeptides, one or more nucleic acids and one or more small molecules, y) one or more polypeptides, one or more nucleic acids and one or more metal-containing compounds, z) one or more polypeptides, one or more lipids and one or more carbohydrates, aa) one or more polypeptides, one or more lipids and one or more small molecules, ab) one or more polypeptides, one or more lipids and one or more metal-containing compounds, ac) one or more polypeptides, one or more carbohydrates and one or more small molecules, ad) one or more polypeptides, one or more carbohydrates and one or more metal-containing compounds, ae) one or more polypeptides, one or more small molecules and one or more metal-containing compounds, af) one or more nucleic acids, one or more lipids, and one or more carbohydrates, ag) one or more nucleic acids, one or more lipids, and one or more small molecules, ah) one or more nucleic acids, one or more lipids, and one or more metal-containing compounds, ai) one or more nucleic acids, one or more carbohydrates, and one or more small molecules, aj) one or more nucleic acids, one or more carbohydrates, and one or more metal-containing compounds, ak) one or more nucleic acids, one or more small molecules, and one or more metal-containing compounds, al) one or more lipids, one or more carbohydrates and one or more small molecules, am) one or more lipids, one or more carbohydrates and one or more metal-containing compounds, an) one or more lipids, one or more small molecules and one or more metal-containing compounds, ao) one or more carbohydrates, one or more small molecules and one or more metal-containing compounds, ap) one or more polypeptides, one or more nucleic acids, one or more lipids, and one or more carbohydrates, aq) one or more polypeptides, one or more nucleic acids, one or more lipids, and one or more small molecules, ar) one or more polypeptides, one or more nucleic acids, one or more lipids, and one or more metal-containing compounds, as) one or more polypeptides, one or more nucleic acids, one or more carbohydrates, and one or more small molecules, at) one or more polypeptides, one or more nucleic acids, one or more carbohydrates, and one or more metal-containing compounds, au) one or more polypeptides, one or more nucleic acids, one or more small molecules, and one or more metal-containing compounds, av) one or more polypeptides, one or more lipids, one or more carbohydrates, and one or more small molecules, aw) one or more polypeptides, one or more lipids, one or more carbohydrates, and one or more metal-containing compounds, ax) one or more polypeptides, one or more lipids, one or more small molecules, and one or more metal-containing compounds, ay) one or more polypeptides, one or more carbohydrates, one or more small molecules, and one or more metal-containing compounds, az) one or more nucleic acids, one or more lipids, one or more carbohydrates, and one or more small molecules, ba) one or more nucleic acids, one or more lipids, one or more carbohydrates, and one or more metal-containing compounds, bb) one or more nucleic acids, one or more lipids, one or more small molecules, and one or more metal-containing compounds, be) one or more nucleic acids, one or more carbohydrates, one or more small molecules, and one or more metal-containing compounds, bd) one or more lipids, one or more carbohydrates, one or more small molecules, and one or more metal-containing compounds, be) one or more polypeptides, one or more nucleic acids, one or more lipids, one or more carbohydrates, and one or more small molecules, bf) one or more polypeptides, one or more nucleic acids, one or more lipids, one or more carbohydrates, and one or more metal-containing compounds, bg) one or more polypeptides, one or more nucleic acids, one or more lipids, one or more small molecules, and one or more metal-containing compounds, bh) one or more polypeptides, one or more nucleic acids, one or more carbohydrates, one or more small molecules, and one or more metal-containing compounds, bi) one or more polypeptides, one or more lipids, one or more carbohydrates, one or more small molecules, and one or more metal-containing compounds, bj) one or more nucleic acids, one or more lipids, one or more carbohydrates, one or more small molecules, and one or more metal-containing compounds, or bk) one or more polypeptides, one or more nucleic acids, one or more lipids, one or more carbohydrates, one or more small molecules, and one or more metal-containing compounds.
37. The method of any one of embodiments 1-36, wherein the complex comprises an antibody.
38. The method of any one of embodiments 1-36, wherein the complex comprises one or more transcription factors.
39. The method of any one of embodiments 1-36, wherein the complex comprises a ribosome and an mRNA.
40. The method of any one of embodiments 1-36, wherein the complex comprises a proteasome, a holoenzyme, an RNA polymerase, a DNA polymerase, a spliceosome, a vault cytoplasmic ribonucleoprotein, a small nuclear ribonucleic protein (snRNP), a telomerase, a nucleosome, a death signaling complex (DISC), a mammalian target of rapamycin complex 1 (mTORC1), a mammalian target of rapamycin complex 2 (mTORC2), or a class I phosphoinositide 3 kinase (Class I PI3K), RNA-induced silencing complex (RISC), histone-DNA complex, toll-like receptor (TLR)-agonist complex, transposase/transposon complex, tRNA ribosome complex, polypeptide-protease complex, or an enzyme-substrate complex.
41. The method of any one of embodiments 1-40, wherein the cell suspension is contacted with the complex after the cell suspension passes through the constriction.
42. The method of any one of embodiments 1-40, wherein the cell suspension is contacted with the complex before the cell suspension passes through the constriction.
43. The method of any one of embodiments 1-40, wherein the cell suspension is contacted with the complex at the same time the cell suspension passes through the constriction.
44. The method of any one of embodiments 1-42, wherein the complex is formed prior to contact with the cell suspension.
45. The method of embodiment 44, wherein the complex is formed about 1 minute, about 5 minutes, about 10 minutes, about 15 minutes, about 30 minutes, about 45 minutes, about 1 hour, about 2 hours, about 3 hours, or about 6 hours prior to contact with the cell suspension.
46. The method of any one of embodiments 1-45, wherein the complex is purified prior to contact with the cell suspension.
47. The method of embodiment 40, wherein the complex is formed in the cell suspension.
48. The method of embodiment 47, wherein one or more of the molecules of the complex are purified prior to contact with the cell suspension.
49. The method of any one of embodiments 1-48, wherein the cell suspension comprises a mixed cell population.
50. The method of any one of embodiments 1-48, wherein the cell suspension comprises a purified cell population.
51. The method of any one of embodiments 1-48, wherein the cell suspension comprises prokaryotic or eukaryotic cells.
52. The method of any one of embodiments 1-51, wherein the cell suspension comprises bacterial cells, archael cells, yeast cells, fungal cells, algal cells, plant cells or animal cells.
53. The method of any one of embodiments 1-52, wherein the cell suspension comprises vertebrate cells.
54. The method of any one of embodiments 1-53, wherein the cell suspension comprises mammalian cells.
55. The method of any one of embodiments 1-54, wherein the cell suspension comprises human cells.
56. The method of any one of embodiments 1-55, wherein the constriction is contained within a microfluidic channel.
57. The method of any one of embodiments 1-55, wherein the constriction is a pore or contained within a pore.
58. The method of embodiment 57, wherein the pore is contained in a surface.
59. The method of embodiment 58, wherein the surface is a filter.
60. The method of embodiment 58, wherein the surface is a membrane.
61. The method of any one of embodiments 57-60, wherein the pore size is about 0.4 µm, about 1 µm, about 2 µm, about 3 µm, about 4 µm, about 5 µm, about 6 µm, about 7 µm, about 8 µm, about 9 µm, about 10 µm, about 11 µm, about 12 µm, about 13 µm, or about 14 µm.
62. The method of any one of embodiments 1-61, wherein the constriction size is a function of the cell diameter.
63. The method of any one of embodiment 1-62, wherein the constriction size is about 20%, about 30%, about 40%, about 50%, about 60%, about 70%, about 80%, about 90%, or about 99% of the cell diameter.
64. The method of any one of embodiments 1-63, wherein the constriction has a length of about 30 µm and a width of about 3 µm to about 8 µm.
65. The method of any one of embodiments 1-64, wherein the constriction has a length of about 10 µm and a width of about 3 µm to about 8 µm.
66. The method of any one of embodiments 1-65, wherein the method further comprises the step of contacting the cell suspension and complex with an electric field generated by at least one electrode.
67. A system for delivering a complex of two or more molecules into a cell, the system comprising a microfluidic channel comprising a constriction, a cell suspension comprising the cell, and the complex of two or more molecules; wherein the constriction is configured such that the cell can pass through the constriction wherein the constriction deforms the cell thereby causing a perturbation of the cell such that the complex of two or more molecules enters the cell.
68. A system for delivering a complex of two or more molecules into a cell, the system comprising a surface with pores, a cell suspension comprising the cell, and the complex of two or more molecules; wherein the surface with pores is configured such that the cell can pass through the pore wherein the pore deforms the cell thereby causing a perturbation of the cell such that the complex of two or more molecules enters the cell.
69. The system of embodiment 68, wherein the surface is a filter or a membrane.
70. The system of any one of embodiments 67-69, wherein the system further comprises at least one electrode to generate an electric field.
71. The system of any one of embodiments 67-70, wherein formation of the complex of molecules is reversible.
72. The system of any one of embodiments 67-71, wherein at least two or more molecules of the complex associate by noncovalent interactions.
73. The system of any one of embodiments 67-72, wherein the system is used to deliver a complex comprising two or more molecules into a cell by the method of any one of embodiments 1-66.
74. A cell comprising a complex of two or more molecules, wherein the complex of two or more molecules was delivered into the cell by the method of any one of embodiments 1-66.

### EXAMPLES

### Example 1: Constriction-mediated delivery of complexes

A series of experiments are undertaken in cells to demonstrate constriction-mediated delivery of complexes.

Cultured cells are harvested, counted, washed and resuspended at 10-20 × 10⁶ cells/mL in cell culture media for delivery. Polypeptide-nucleic acid complexes are assembled in vitro prior to contact with cells. Complexing conditions, including complex concentration, solution osmolarity, salt concentration, temperature, pH, serum and surfactant content, and viscosity are optimized to ensure that the formed complexes remain in a stable, intact form until post-delivery to the cells. Cell suspensions are passed through two different microfluidic chips, 10-6 or 10-7 chip at pressures of 60 and 90psi. The chips have constrictions of the same width (4 microns) but have two different constriction lengths (30 vs. 10 microns). After passing through the constriction, the cells are incubated with the pre-assembled complexes to allow for delivery of the complexes to the cells. Cellular incubation conditions, including solution osmolarity, salt concentration, temperature, pH, serum and surfactant content, cell and complex concentration, and viscosity are optimized to ensure that the complexes remain in a stable, intact form until post-delivery to the cells. In some examples, complexes are co-delivered with a 3kDa-Cascade Blue Dextran (0.15mg/mL) as a proxy for delivery efficiency.

At 48 hours post-delivery, a FACS based readout is used to determine delivery of the complexes. As a control for endocytosis, cells that did not pass through a constriction are incubated with the pre-assembled complexes for the same time as cells subjected to the constriction-mediated procedure.

### Example 2: Constriction-mediated delivery of complexes to T cells

A series of experiments are undertaken in unstimulated human T cells to demonstrate constriction-mediated delivery of complexes.

Fresh PBMCs are isolated from human blood using a standard Ficoll gradient. Next, T cells are negatively selected (Human T cell enrichment kit (StemCell Technologies)) counted, washed and resuspended at 10-20 × 10⁶ cells/mL in OptiMEM for delivery. Polypeptide-nucleic acid complexes are assembled in vitro prior to cell constriction. Complexing conditions, including complex concentration, solution osmolarity, salt concentration, temperature, pH, serum and surfactant content, and viscosity are optimized to ensure that the formed complexes remain in a stable, intact form until post-delivery to the T cells. T cell suspensions are passed through two different microfluidic chips, 10-4 and 30-4, at pressures of 60 and 90 psi. The chips have constrictions of the same width (4 microns) but have two different constriction lengths (30 vs. 10 microns). After passing through the constriction, the T cells are incubated with the pre-assembled complexes to allow for delivery of the complexes to the cells. Cellular incubation conditions, including solution osmolarity, salt concentration, temperature, pH, serum and surfactant content, cell and complex concentration, and viscosity are optimized to ensure that the complexes remain in a stable, intact form until post-delivery to the T cells. Complexes are co-delivered with a 3kDa-Cascade Blue Dextran (0.15mg/mL) as a proxy for delivery efficiency.

At 48 hours post-delivery, a FACS based readout is used to determine delivery of the complexes. As a control for endocytosis, T cells that did not pass through a constriction are incubated with the pre-assembled complexes for the same time as cells subjected to the constriction-mediated procedure.

### Example 3: Constriction-mediated delivery of protein/protein/nucleic acid complexes to HEK293 cells

In this study, constriction-mediated delivery of a protein/protein/nucleic acid complex was evaluated. The complex contained a ribonucleoprotein (RNP) complex containing CAS9 protein and guide RNA (gRNA) designed to knockdown the *B2M* locus complexed *in vitro* with fluorescently labeled anti-CAS9 IgG antibody.

First, the RNP complex was formed by combining 10 µg of recombinant CAS9 protein (PNA Bio) with a 2.5 molar excess of unmodified gRNA (PNA Bio) designed to specifically target the *B2M* locus, followed by incubation on ice for 20 minutes. Next, anti-CAS9 IgG antibody (Cell Signaling Technology) was complexed at room temperature with the RNP complex at a 1:11 molar ratio of antibody:RNP. HEK293 cells were counted, washed, and resuspended at 10-20 × 10⁶ cells/mL in OptiMEM containing the antibody/CAS9/gRNA complex. The antibody/CAS9/gRNA complex was delivered to the cells by passing the cell suspension through a microfluidic chip having a constriction length of 10 microns and a constriction width of 7 microns at 90 psi. 3kDa-Cascade Blue dextran was co-delivered with the complex by inclusion in the cell suspension at 0.15mg/mL and used as an internal standard for uniformity of delivery conditions between the samples. The experimental controls included a sample for which the cells were exposed to the antibody/CAS9/gRNA complex without constriction for the duration of the treatment (endocytosis control), a sample where RNP complex alone was subjected to constriction-mediated delivery, and a sample where antibody alone was subjected to constriction-mediated delivery. All samples included the 3kDa-Cascade Blue dextran internal standard.

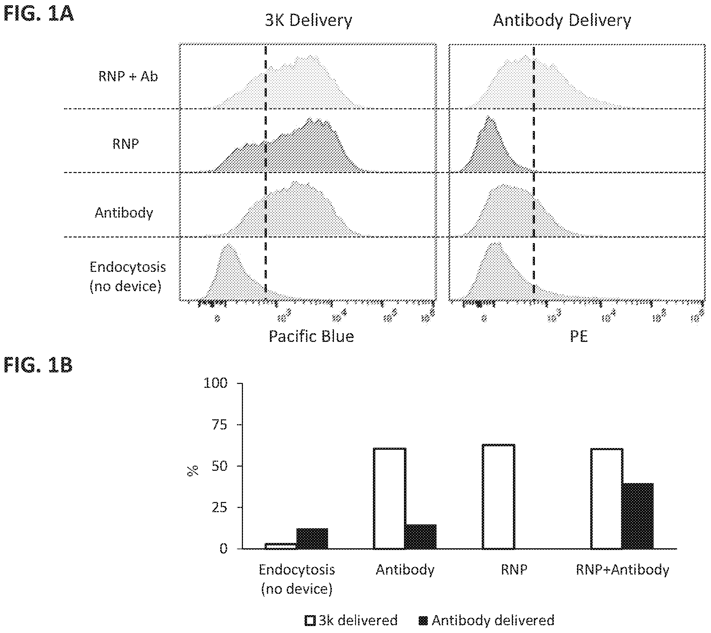

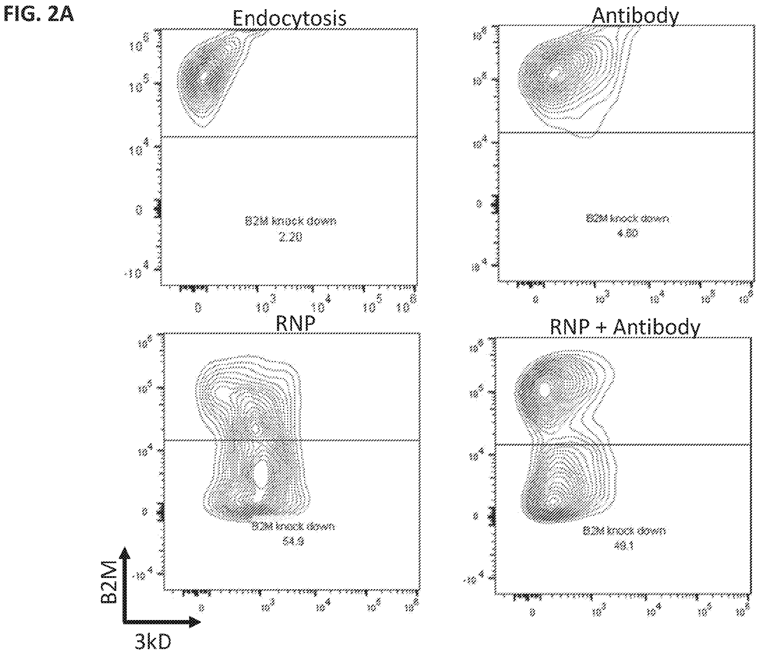

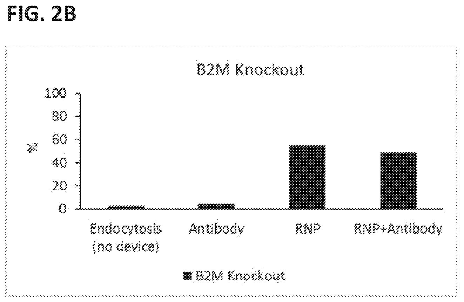

FACS analysis was conducted at 24 hours and 6 days after passing the cell suspensions through the microfluidic chip to assess delivery and B2M knockdown, respectively. At 24 hours, the cells were assessed by FACS analysis to determine positivity for 3kDa-Cascade Blue dextran and the fluorescently labeled anti-CAS9 antibody. The percentage of cells positive for the 3kDa-Cascade Blue dextran internal standard was similar across all squeezed samples (60.5%, 62.7% and 60.3%) (FIGS. 1A and 1B), indicating that the delivery conditions during constriction were consistent for each sample and allowing for comparison of the delivery of the different cargoes. We found that the delivery of the antibody was higher (39.6% vs. 14.7% anti-CAS9 antibody⁺) when it was pre-complexed with the RNP complex as opposed to when it was delivered alone (FIGS. 1A and 1B). This finding was unexpected as the complex is larger than the antibody alone. At day 6, the cells were stained for B2M and FACS analysis was performed to assess the level of B2M protein knockdown attributed to the RNP. The RNP alone and antibody/CAS9/gRNA complex conditions both showed knockdown of B2M, indicating successful delivery of functional complexes. The editing was slightly higher in the condition with the RNP alone than when complexed with the antibody (54.9% vs. 49.1% B2M⁻) (FIG. 2).

### Example 4: Constriction-mediated delivery of protein/small molecule complexes to HeLa cells

In this study, constriction-mediated delivery of a protein/small molecule complex was evaluated. The complex contained fluorescently labeled streptavidin and fluorescently labeled biotin.

A 2 µM solution of Pacific Blue-labeled streptavidin in OptiMEM was combined with varying concentrations of FITC-labeled biotin in OptiMEM (2, 8, or 16 µM) to achieve biotin:streptavidin molar ratios of 1:1, 4:1 and 8:1, respectively, at 0°C and kept on ice for 1 hour. HeLa cells were counted, washed, and resuspended at 1 × 10⁶ cells/mL in OptiMEM. The streptavidin-biotin complex was delivered to the cells by passing the cell suspension through a microfluidic chip having a constriction length of 10 microns and a constriction width of 7 microns at 90 psi. 3kDa-AlexaFluor 680 dextran was co-delivered with the complex by inclusion in the cell suspension at 0.1 mg/mL and used as an internal standard for uniformity of delivery conditions between the samples. The experimental controls included samples for which the cells were exposed to the streptavidin-biotin complexes (all molar ratios) without constriction for the duration of the treatment (endocytosis controls), a sample where the streptavidin and biotin (1:1 molar ratio) were not pre-incubated prior to constriction-mediated delivery, a sample where streptavidin alone (2 µM) was subjected to constriction-mediated delivery, and samples where biotin alone (2 µM, 8 µM, and 16 µM) was subjected to constriction-mediated delivery. All samples included the 3kDa-AlexaFluor 680 dextran internal standard.

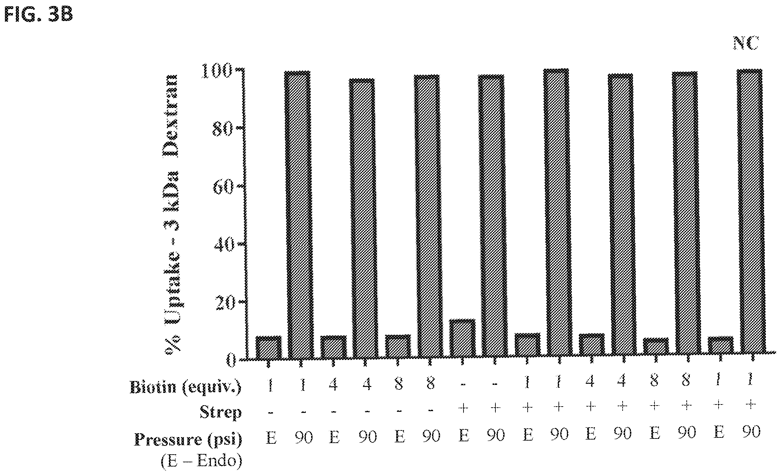

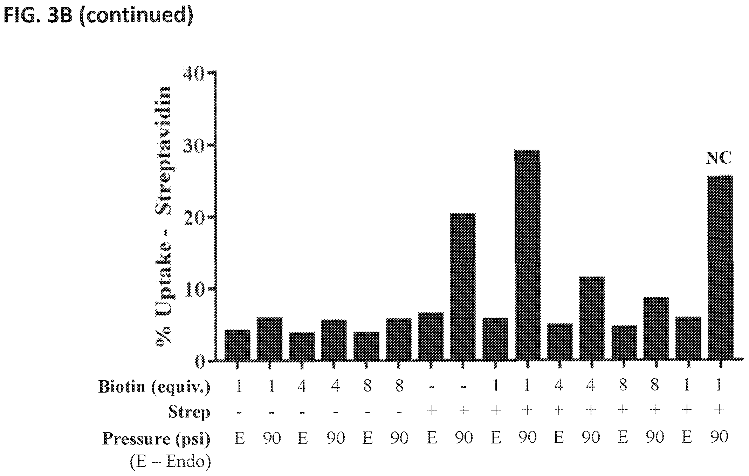

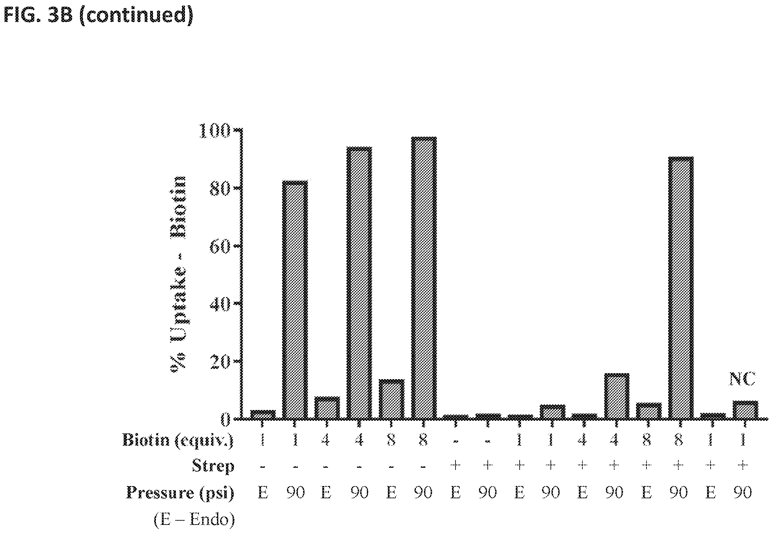

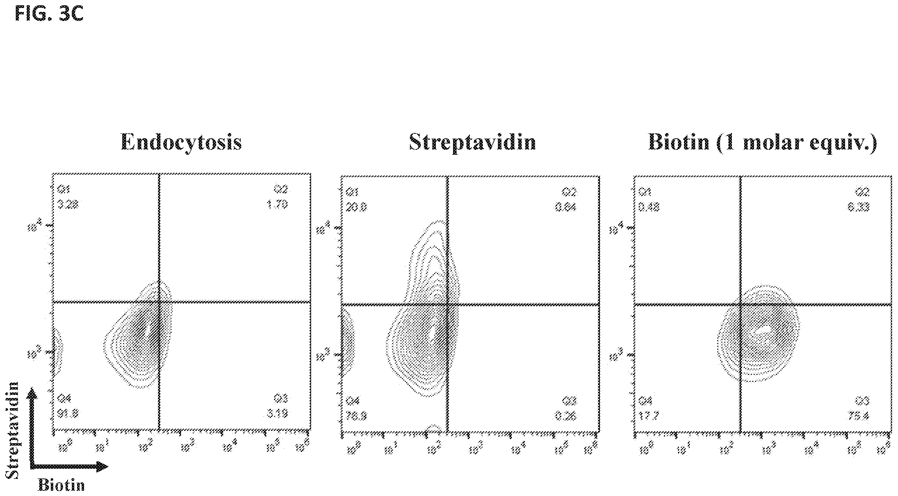

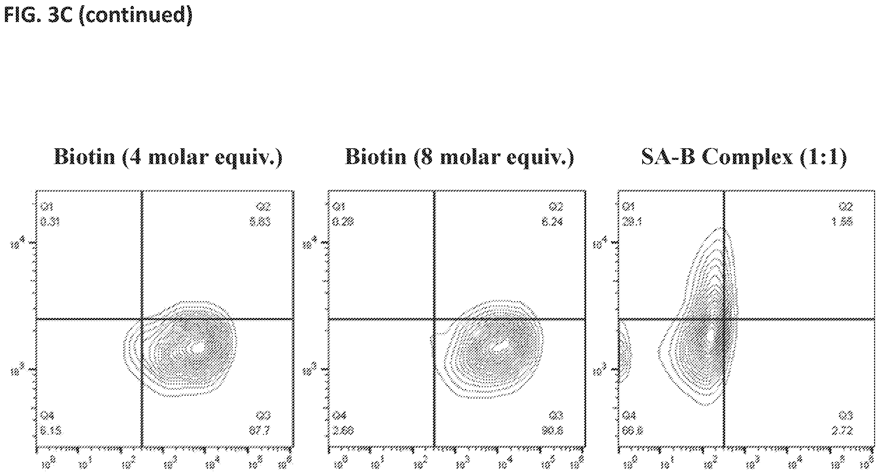

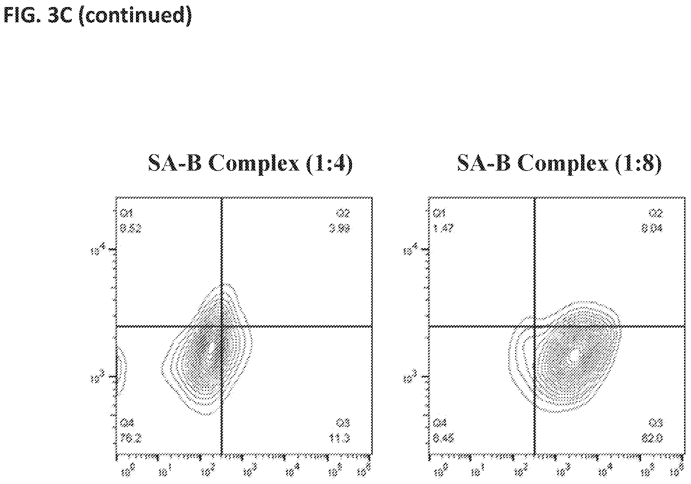

FACS analysis was conducted immediately after passing the cell suspensions through the microfluidic chip to assess complex and dextran delivery by determining positivity for 3kDa-AlexaFluor 680 dextran, streptavidin, and biotin. The percentage of cells positive for the 3kDa-AlexaFluor 680 dextran internal standard was similar across all squeezed samples (76-86%, FIGS. 3A and 3B), allowing for comparison of the delivery of the different cargoes. It was observed that streptavidin delivery in the streptavidin alone condition (20.1% streptavidin⁺) and 1:1 streptavidin-biotin complex condition (28.9% streptavidin⁺, FIGS. 3A, 3B, and 3C) was similar, indicating that streptavidin was able to enter the cells under the tested constriction conditions. Biotin uptake occurred in a dosedependent fashion (81-97% biotin⁺, FIGS. 3A, 3B, and 3C) when administered alone, but the addition of streptavidin caused an almost complete loss of biotin fluorescent signal at 1:1 and 4:1 biotin:streptavidin molar ratios (4-15% biotin⁺). This is indicative of complex formation, as biotin-fluorescein fluorescence is known to be quenched once it is bound to streptavidin (Kada et al., Biochim. Biophys. Acta, 1427 (1999) 44-48), and streptavidin can bind up to 4 molecules of biotin per molecule of streptavidin. Introduction of excess biotin (8:1 biotin:streptavidin molar ratio) increased biotin-related fluorescence due to the presence of non-complexed intracellular biotin. Interestingly, the streptavidin signal decreased with increasing relative concentrations of biotin, possibly due to cross-talk with the streptavidin fluorophore. The 1:1 "non-complex" control (NC) where the reagents were not pre-incubated showed similar levels of delivery compared to the 1:1 pre-complexed sample for both streptavidin and biotin, supporting very rapid binding of the complex or intracellular complexation (FIGS. 3A, 3B, and 3C). Taken together, these results indicate that complex formation occurred rapidly, with delivery of the complex by passage through the microfluidic device, as indicated by the intracellular streptavidin fluorescence coincident with the wellcharacterized quenching of biotin fluorescence that is only observed during complexation.

In another study, constriction-mediated delivery of a protein/small molecule complex containing fluorescently labeled streptavidin and phalloidin-conjugated biotin was evaluated.

200 pmol of Pacific Blue-conjugated streptavidin (SA) was pre-complexed with 200 pmol of phalloidin-conjugated biotin (B-Phall). The streptavidin-biotin complexes were incubated on ice for 40 minutes (1:1 ratio). HeLa cells were counted, washed, and resuspended at 2-10 × 10⁶ cells/mL in OptiMEM containing the complex at 2 µM. The streptavidin-biotin complex was delivered to the cells by passing the cell suspension through a microfluidic chip having a constriction length of 10 microns and a constriction width of 7 microns at 90 psi. 3kDa-AlexaFluor 680 dextran was co-delivered with the complex by inclusion in the cell suspension at 0.1 mg/mL and used as an internal standard for uniformity of delivery conditions between the samples. The experimental controls included a sample for which the cells were exposed to the streptavidin-biotin complex without constriction for the duration of the treatment (endocytosis control), a sample where SA alone was subjected to constriction-mediated delivery, and a sample where B-Phall alone was subjected to constriction-mediated delivery. All samples included the 3kDa-AlexaFluor 680 dextran internal standard.

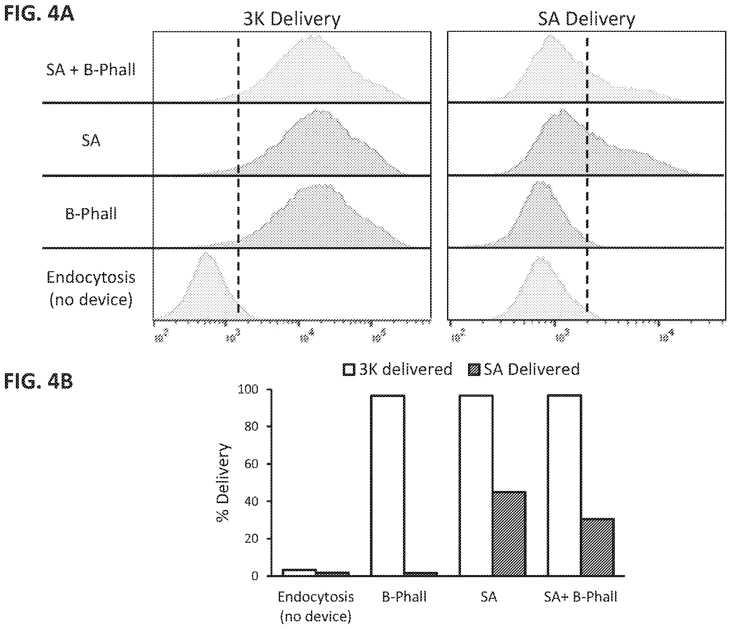

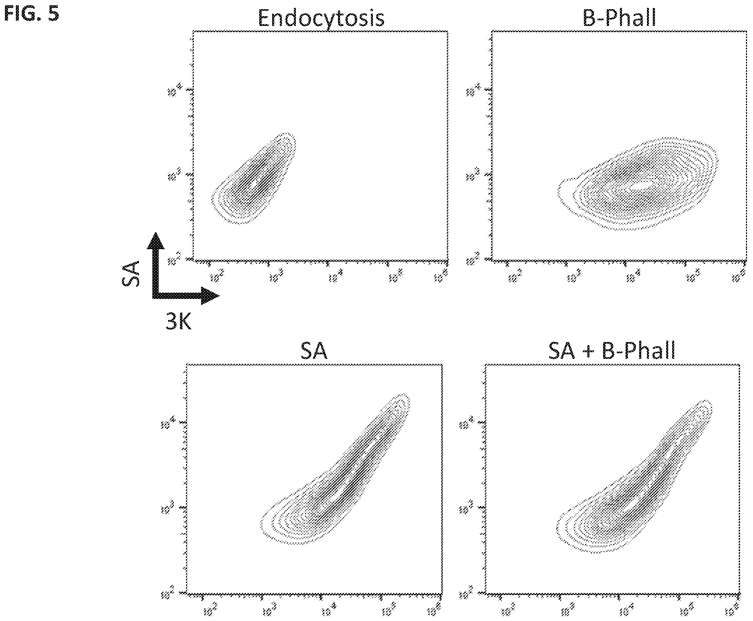

Flow cytometry analysis was conducted immediately after passing the cell suspensions through the microfluidic chip to assess complex and dextran delivery by determining positivity for 3kDa-AlexaFluor 680 dextran and SA. The percentage of cells positive for the 3kDa-AlexaFluor 680 dextran internal standard was similar across all squeezed samples (96.6%, 96.7%, and 96.8%) (FIGS. 4A and 4B), allowing for comparison of the delivery of the different cargoes. We found that the delivery of the SA was lower when it was pre-complexed with the B-Phall as compared to when it was delivered alone (30.5% vs. 44.9% SA⁺, FIGS. 4A and 4B). In samples with SA, we found that 3kDa-AlexaFluor 680 dextran delivery correlated with SA delivery (FIG. 5).

### Example 5: Constriction-mediated delivery of protein/protein complexes to T cells

In this study, constriction-mediated delivery of protein/protein complexes was evaluated. The complex contained HPV16 E7 synthetic long peptide (SLP) and mouse serum albumin (MSA).

HPV 16 E7 SLP was resuspended in water at a concentration of 2.55 µM. MSA was resuspended in RPMI (Thermo) at 132.2 µM. To form complexes, two-fold the desired final concentration of SLP was incubated for 10 minutes at room temperature with 40 µM MSA in RPMI. T cells were isolated from C57BL/6 mice using the EasySep Mouse Pan T cell isolation kit (StemCell), washed, counted, and resuspended in RPMI at 20 × 10⁶ cells/mL for immediate use. Uncomplexed SLP or SLP/MSA complex was added directly to murine T cells at the indicated concentrations (5 µM, 10 µM, or 20 µM) and delivered to the cells by passing the cell suspension through a microfluidic chip having a constriction length of 10 microns and a constriction width of 3 microns at 90 psi. 3kDa-Cascade Blue dextran was co-delivered with the complex by inclusion in the cell suspension at 0.15 mg/mL and used as an internal standard for uniformity of delivery conditions between the samples. The experimental controls included samples for which the cells were exposed to the SLP or SLP/MSA complex without constriction for the duration of the treatment (endocytosis controls), and a vehicle alone sample including only RPMI + water. All samples included the 3kDa-Cascade Blue dextran internal standard.

*In vivo* studies were carried out with C57BL/6 mice primed on Day 0 with intravenous (i.v.) administration of a) 5 × 10⁶ T cells subjected to constriction-mediated delivery of either SLP or SLP/MSA; b) 5 × 10⁶ T cells incubated with SLP or SLP/MSA without constriction as controls for endocytosis and/or nonspecific binding; or c) 5 × 10⁶ T cells subjected to constriction-mediated delivery with vehicle alone (no antigen). On Day 6, mice were bled and levels of E7-specific cytotoxic T lymphocytes (CTLs) circulating in the blood were determined using iTAg Tetramer/PE - H-2 Db HPV 16 E7 tetramer (MBL). Flow cytometry analysis was performed using FlowJo.

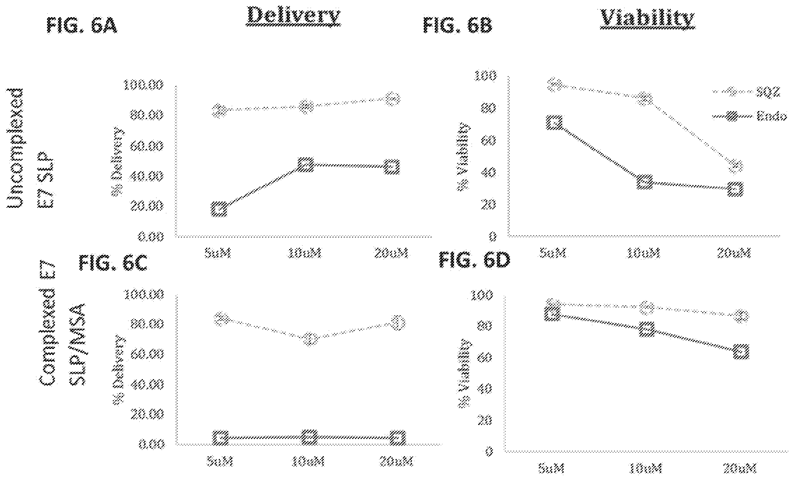

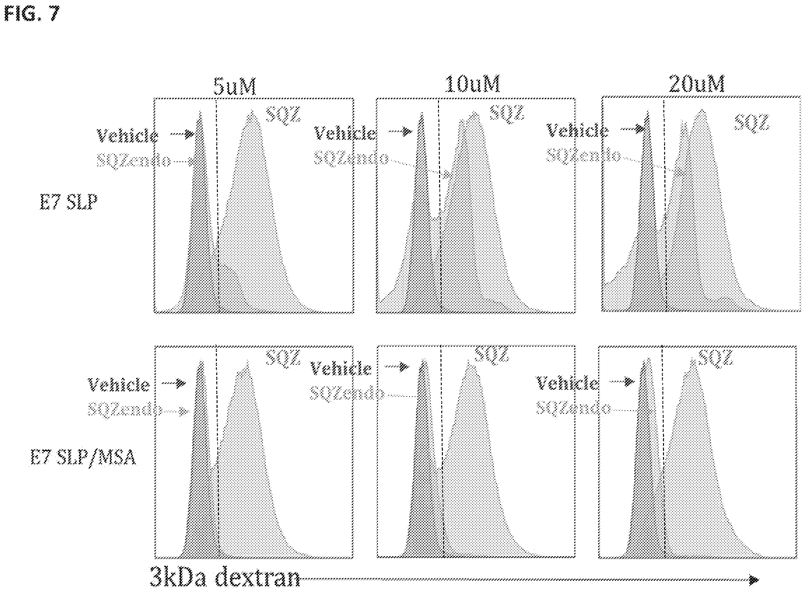

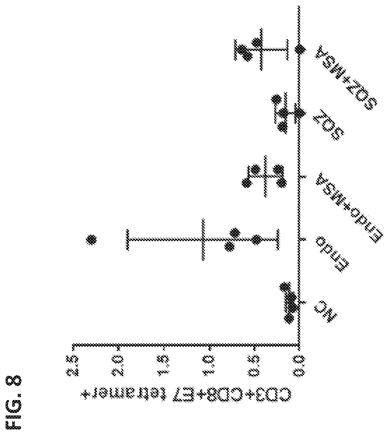

FACS analysis was carried out immediately following passage through the microfluidic chip to assess dextran delivery by determining positivity for 3kDa-Cascade Blue dextran. FIG. 6A shows that without constriction, the fraction of dextran⁺ cells was relatively high with SLA alone, particularly at higher SLP concentrations. We also saw that without constriction, the viability (as determined by propidium iodide staining) of the T cells at higher SLP concentrations was significantly decreased (FIG. 6B). This indicates that incubation with SLP alone led to high background and high toxicity. When the SLP was complexed with MSA, the control signal without constriction was significantly reduced (FIG. 6C). We also found that the toxicity of the complex (both with and without constriction) was significantly less than for SLP alone at the same concentration (FIGS. 6B and 6D). Together this indicates that the formation of the complex significantly and unexpectedly altered the delivery dynamic and resultant viability. FIG. 7 shows representative flow cytometry plots for the various conditions. FIG. 8 shows the endogenous CD8 T-cell response as measured by tetramer staining for E7-specific CTLs six days after the treated T cells were introduced back into the mouse. As seen by tetramer staining, T cells incubated with SLP/MSA complexes under control conditions without constriction (Endo + MSA) resulted in greatly reduced levels of CD8 responses as compared to T cells incubated with SLP alone without constriction (Endo). Unexpectedly, given these results, T cells incubated with SLP/MSA complexes with constriction (SQZ + MSA) resulted in increased levels of CD8 responses as compared to T cells incubated with SLP alone with constriction (SQZ).

## Claims

1. A method for delivering a complex of two or more molecules into a cell, the method comprising: passing a cell suspension through a constriction, wherein said constriction deforms the cell, thereby causing a perturbation of the cell such that the complex of molecules enters the cell, wherein said cell suspension is contacted with the complex of molecules, wherein the complex of molecules comprises one or more polypeptides.

2. The method of claim 1, wherein formation of the complex of molecules is reversible.

3. The method of claim 1 or 2, wherein at least two or more molecules of the complex associate by noncovalent interactions.

4. The method of any one of claims 1-3, wherein the complex is formed in the cell suspension, and/or has a half-life in the cell suspension of about 1 minute to about 48 hours.

5. The method of any one of claims 1 to 4, wherein the complex dissociates:
a) in the presence of a detergent at a concentration of about 0.1% (w/v) to about 10% (w/v);
b) at a temperature greater than the temperature at which the cell suspension is contacted with the complex of molecules;
c) at an ionic strength greater than the ionic strength at which the cell suspension is contacted with the complex of molecules;
d) at an ionic strength less than the ionic strength at which the cell suspension is contacted with the complex of molecules;
e) at an osmolarity greater than the ionic strength at which the cell suspension is contacted with the complex of molecules;
f) at an osmolarity less than the osmolarity at which the cell suspension is contacted with the complex of molecules,
g) at a pH greater or lower than the pH at which the cell suspension is contacted with the complex of molecules; or
h) at a shear force of about 10 kPa to about 100 kPa.

6. The method of any one of claims 1-5, wherein the cell suspension is contacted with the complex at the same time the cell suspension passes through the constriction, and/or after the cell suspension passes through the constriction.

7. The method of any one of claims 1-6, wherein one or more of the molecules of the complex are purified prior to contact with the cell suspension.

8. The method of any one of claims 1-7, wherein, and the constriction is a) contained within a microfluidic channel, or b) a pore or contained within a pore.

9. The method of claim 8, wherein the pore size is about 0.4 µm, about 1 µm, about 2 µm, about 3 µm, about 4 µm, about 5 µm, about 6 µm, about 7 µm, about 8 µm, about 9 µm, about 10 µm, about 11 µm, about 12 µm, about 13 µm, or about 14 µm.

10. The method of any one of claims 1-9, wherein a size of the constriction is a function of the cell diameter.

11. The method of any one of claims 1-10, further comprising co-delivering a fluorophore- and/or a fluorescently-labeled molecule.

12. The method of claim 11, wherein the fluorophore- and/or the fluorescently-labeled molecule is bound to the complex, and/or is a proxy for delivery efficiency.

13. The method of any one of claims 1-12, wherein the complex of two or more molecules is a polypeptide-nucleic acid complex.

14. The method of any one of claim 1-13, wherein the cell is comprised in a population of desired cells, and the population of desired cells are isolated from a sample.

15. The method of any one of claims 1-14, wherein a viscosity of the cell suspension ranges between about 0.89 cP and about 4.0 cP.
